# EUROPEAN PATENT APPLICATION

(11) **EP 4 458 282 A1**
(43) Date of publication of application: **06.11.2024**
(21) Application number: 23743259.6
(22) Date of filing: 18.01.2023
(51) Int. Cl.: A61B 17/135

(54) **HEMOSTASIS DEVICE**

(30) Priority: 19.01.2022 JP 2022006151
(71) Applicant: Terumo Kabushiki Kaisha, Tokyo 151-0072 (JP)
(72) Inventor: YAMASHITA, Koki, Fujinomiya-shi, Shizuoka 418-0015 (JP); OUCHI, Tatsuya, Fujinomiya-shi, Shizuoka 418-0015 (JP); KAWAMURA, Tomoya, Fujinomiya-shi, Shizuoka 418-0015 (JP); HIDAKA, Yuna, Fujinomiya-shi, Shizuoka 418-0015 (JP)
(74) Representative: Casalonga
(86) International application number: PCT/JP2023/001253
(87) International publication number: WO 2023/140265

(57) **Abstract**

A hemostatic device capable of enhancing a degree of freedom of a position at which the hemostatic device is attached to a body of a patient and preventing band bodies from inadvertently sliding and moving during hemostasis is provided.

A support member 200 provided in a hemostatic device 10 includes a first region 210 in which a pressing portion 300 is disposed, and a second region 220 located outside the first region and configured such that a first band body 410 and a second band body 420 are connectable. The second region includes a first hole portion 240 configured such that the first band body is connectable, and a second hole portion 250 located at a position facing the first hole portion across the pressing portion and configured such that the second band body is connectable. The first hole portion and the second hole portion include a plurality of protrusions 280 protruding toward the pressing portion.

## Description

### Technical Field

The present invention relates to a hemostatic device.

### Background Art

As one of catheter procedures, there is known a procedure in which various medical elongated bodies are introduced into a blood vessel through a puncture site formed by puncturing a blood vessel of the arm or the hand of a patient to perform a procedure or a therapy at a lesion site. For example, Patent Literature 1 discloses a hemostatic device for stopping bleeding at a puncture site formed to enable access to a blood vessel (including a distal radial artery) running in the hand.

The hemostatic device of Patent Literature 1 includes a pressing portion including a balloon for applying a compressive force to a puncture site formed on the hand of a patient, a plurality of band bodies for fixing the pressing portion to the hand of the patient, and a support member for connecting the pressing portion and the plurality of band bodies. In addition, the plurality of band bodies include a band body for wrapping disposed to be wrapped along an external periphery of the hand, and a band body for finger hooking disposed at an interdigital part located between adjacent fingers of the hand.

### Citation List

### Patent Literature

Patent Literature 1: US 2019/0133602 A

### Summary of Invention

### Technical Problem

An operator such as a doctor (hereinafter, referred to as an "operator") disposes a pressing portion so as to overlap a puncture site formed in the hand of a patient when stopping bleeding at the puncture site using the hemostatic device of Patent Literature 1. The operator wraps a band body for wrapping along an external periphery of the hand and further disposes a band body for finger hooking between the thumb and the index finger. By fixing the hemostatic device using each band body in a state where the pressing portion is disposed at the puncture site formed on the hand of the patient and in a peripheral portion of the puncture site, the operator can prevent the pressing portion from being displaced from the puncture site formed on the hand of the patient.

The hemostatic device described in Patent Literature 1 may have the following problems.

A position of the blood vessel running on the hand may be different for each patient. In addition, there are various differences in a size of the hand of the patient depending on a difference in physique for each patient. The position of the blood vessel, the size of the hand, and the like, are different for each patient, and thus, the operator may form puncture sites at different positions of the hand of the patient each time a procedure is performed. For example, the operator may form a puncture site in an anatomical snuff box located on the dorsal side of the hand or may form a puncture site at a position on a peripheral side of the snuff box (on the fingertip side of the hand with respect to the snuff box).

The hemostatic device of Patent Literature 1 is not intended to be used for puncture sites formed at different positions of the hand. Thus, a connection position at which the band body for wrapping and the support member are connected is always fixed. Thus, in the hemostatic device of Patent Literature 1, a degree of freedom of a position at which the band body for wrapping is attached to the hand is low.

As a measure against the above problem, for example, it is conceivable to form a hole portion extending along a periphery of the pressing portion in the support member and connect the band body for wrapping to the support member via the hole portion. In a case of such a configuration, the band body for wrapping can slide and move along an extending direction of the hole portion in a state where the band body for wrapping is connected to the hole portion. Thus, in the hemostatic device, the position of the band body for wrapping can be adjusted when the hemostatic device is attached to the hand of the patient, and thus, a degree of freedom of the position at which the hemostatic device is attached to the hand of the patient becomes higher.

However, in a case where the band body for wrapping is configured to be able to slide and move along the hole portion formed in the support member as described above, the hemostatic device may have the following problems.

When the patient makes some movement in a state where the hemostatic device is attached, if the hemostatic device touches a surrounding article, or the like, the band body for wrapping may move, and the support member and the pressing portion connected to the support member may tilt or move. In the hemostatic device, if the pressing portion tilts or moves as described above during hemostasis, a compressing direction of the pressing portion is deviated from the puncture site where bleeding is to be stopped. This makes it difficult for the hemostatic device to appropriately apply a compressive force to the puncture site.

In view of the above problems, it is an object of the present invention to provide a hemostatic device capable of enhancing a degree of freedom of a position at which the hemostatic device is attached to the body of a patient and preventing band bodies from inadvertently sliding and moving during hemostasis using the hemostatic device.

### Solution to Problem

A hemostatic device according to the present invention includes a pressing member configured to compress a puncture site formed on a patient, a first band body configured to be connectable to the pressing member, and a second band body configured to be connectable to the pressing member, in which the pressing member includes a support member and a pressing portion fixed to the support member and configured to compress the puncture site, the support member includes a first region in which the pressing portion is disposed and a second region located outside the first region and configured such that the first band body and the second band body are connectable, the second region includes a first hole portion configured such that the first band body is connectable, and a second hole portion located at a position facing the first hole portion across the pressing portion and configured such that the second band body is connectable, and the first hole portion and the second hole portion have a plurality of protrusions protruding toward the pressing portion.

### Advantageous Effects of Invention

In the hemostatic device of the present invention, the first band body connected to the first hole portion formed in the second region of the support member provided in the pressing member can slide and move along the first hole portion, and the second band body connected to the second hole portion formed in the second region of the support member can slide and move along the second hole portion. Thus, in the hemostatic device, connection positions at which the first band body and the second band body are connected on the support member can be easily adjusted when the hemostatic device is attached to the body of the patient. As a result, the hemostatic device has a high degree of freedom in a position at which the hemostatic device is attached to the body of the patient. The first hole portion and the second hole portion formed in the support member have a plurality of protrusions protruding toward the pressing portion. When the hemostatic device is attached to the body of the patient, the first band body and the second band body can be disposed so as to be wrapped along part of the body of the patient. In the hemostatic device, if the first band body and the second band body are disposed so as to be wrapped around part of the body of the patient, a tensile force in a direction away from the pressing portion is applied to the first band body and the second band body. As a result, the protrusions located in the first hole portion and the second hole portion bite into the first band body and the second band body. The protrusions restrict sliding movement of the first band body and the second band body as a result of the protrusions biting into the first band body and the second band body. As a result, the hemostatic device can prevent the first band body and the second band body from inadvertently sliding and moving while the pressing portion applies a compressive force to the puncture site.

### Brief Description of Drawings

Fig. 1 is a view illustrating a hemostatic device according to an embodiment, and is a plan view seen from an outer surface side of each band body.
Fig. 2 is a view illustrating the hemostatic device according to the embodiment, and is a plan view seen from an inner surface side of each band body.
Fig. 3 is an enlarged plan view illustrating part of the hemostatic device seen from the outer surface side of each band body.
Fig. 4 is an enlarged plan view illustrating part of the hemostatic device seen from the inner surface side of each band body.
Fig. 5 is an enlarged plan view illustrating part of the hemostatic device seen from the inner surface side of each band body.
Fig. 6 is a cross-sectional view of the hemostatic device taken along an arrow 6A-6A illustrated in Fig. 3, and is a view illustrating a state when a pressing portion inflates.
Fig. 7 is a cross-sectional view of the hemostatic device taken along an arrow 7A-7A illustrated in Fig. 3, and is a view illustrating a state when the pressing portion inflates.
Fig. 8 is a perspective view illustrating a support member.
Fig. 9 is a side view of the support member seen from a direction of an arrow 9A illustrated in Fig. 8.
Fig. 10 is a perspective view illustrating the support member.
Fig. 11 is a plan view illustrating a pressing member.
Fig. 12A is an enlarged plan view illustrating a first hole portion and part of a first band body.
Fig. 12B is an enlarged plan view illustrating a second hole portion and part of a second band body.
Fig. 13 is a cross-sectional view taken along an arrow 13A-13A illustrated in Fig. 12A.
Fig. 14 is a plan view illustrating a state before and after the first band body and the second band body connected to the support member slide and move.
Fig. 15 is a plan view illustrating a state before and after the first band body and the second band body connected to the support member slide and move.
Fig. 16 is a view illustrating the hand (right hand) of a patient for which the hemostatic device is to be used.
Fig. 17 is a view schematically illustrating Use Example 1 of the hemostatic device.
Fig. 18 is a view schematically illustrating Use Example 1 of the hemostatic device.
Fig. 19 is a view schematically illustrating Use Example 1 of the hemostatic device.
Fig. 20 is a partial cross-sectional view taken along an arrow 20A-20A illustrated in Fig. 19.
Fig. 21 is a partial cross-sectional view taken along an arrow 21A-21A illustrated in Fig. 19.
Fig. 22 is a view schematically illustrating Use Example 2 of the hemostatic device.
Fig. 23 is a plan view illustrating a support member according to Modification 1.
Fig. 24 is a plan view illustrating a support member according to Modification 2.
Fig. 25 is a plan view illustrating a support member according to Modification 3.
Fig. 26 is a plan view illustrating a support member according to Modification 4.
Fig. 27 is an enlarged plan view illustrating part of a support member according to Modification 5.
Fig. 28 is a plan view for explaining a use example of a hemostatic device according to a second embodiment.
Fig. 29 is a plan view for explaining a use example of the hemostatic device according to the second embodiment.
Fig. 30 is a partial cross-sectional view taken along an arrow 30A-30A illustrated in Fig. 28.
Fig. 31 is a partial cross-sectional view illustrating a use example of the hemostatic device according to the second embodiment.

### Description of Embodiments

Hereinafter, embodiments of the present invention will be described with reference to the accompanying drawings. The following description does not limit the technical scope or the significance of each term disclosed in the claims. Furthermore, dimensional ratios of the drawings are exaggerated for illustration purpose and may differ from actual ratios.

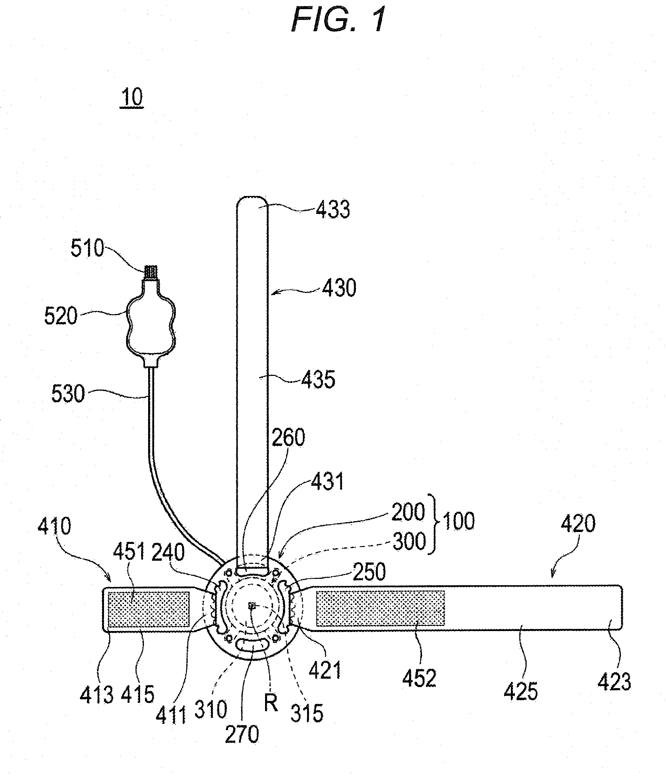

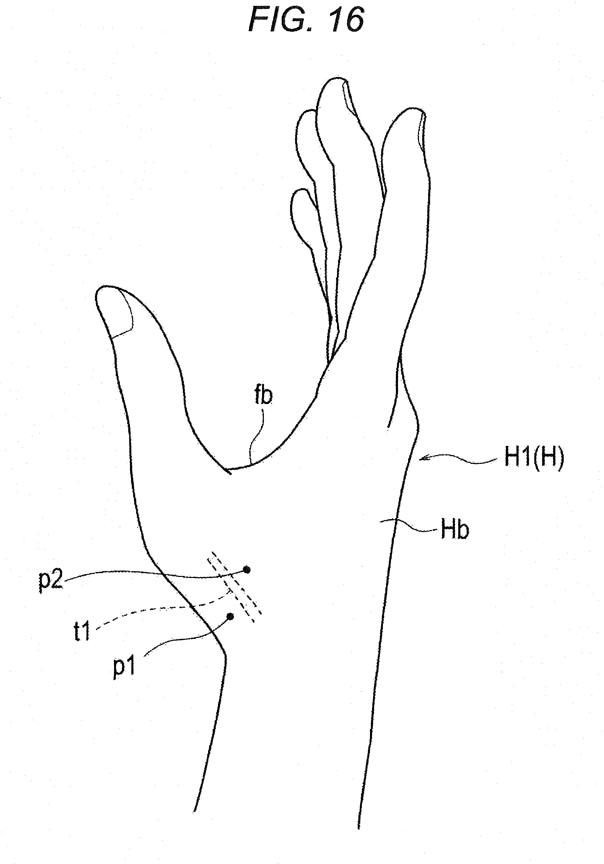

Figs. 1 to 15 are views for explaining a hemostatic device 10 according to the present embodiment, and Figs. 16 to 22 are views for explaining use examples of the hemostatic device 10.

For example, as illustrated in Figs. 16 and 18 to 22, the hemostatic device 10 can be used to stop bleeding at a puncture site (for example, puncture sites p1 and p2 to be described later) formed in the hand H located on a distal side (finger side) of a forearm A of the patient when a sheath tube 610 of an introducer 600 is removed from the puncture site.

A specific position of the puncture site where bleeding is to be stopped by the hemostatic device 10 is not particularly limited, but in the present embodiment, the following first puncture site p1 and second puncture site p2 are exemplified. Note that in the present specification, a structure of each portion of the hemostatic device 10 will be described mainly through an example where the hemostatic device 10 is used to stop bleeding at the first puncture site p1.

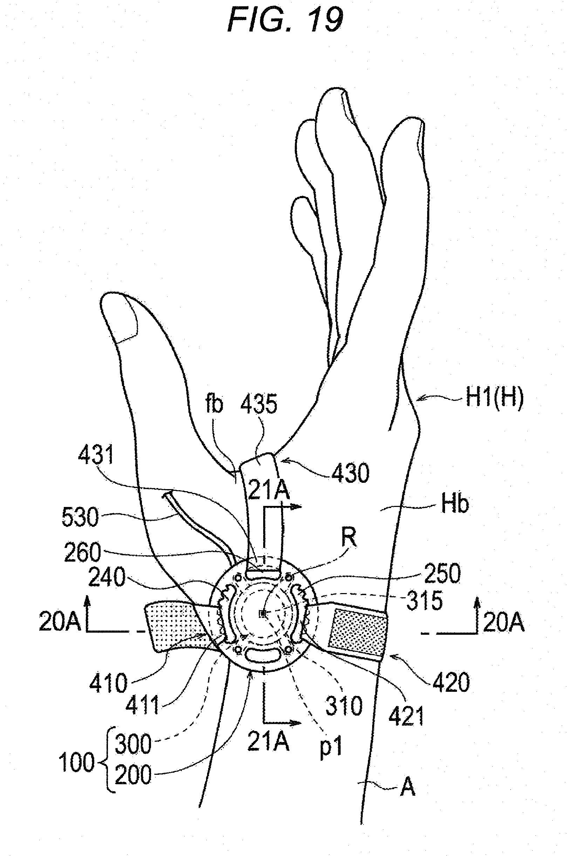

As illustrated in Figs. 16 and 19, the first puncture site p1 is a puncture site formed in the artery B (hereinafter, also referred to as a "blood vessel B") located in the snuff box of the palmar artery running on the dorsal side Hb of the right hand H1 (hand H) located on the distal side of the forearm A of the patient. Note that the snuff box is a cavity of the hand located near the radius when the patient spreads the thumb of the hand H.

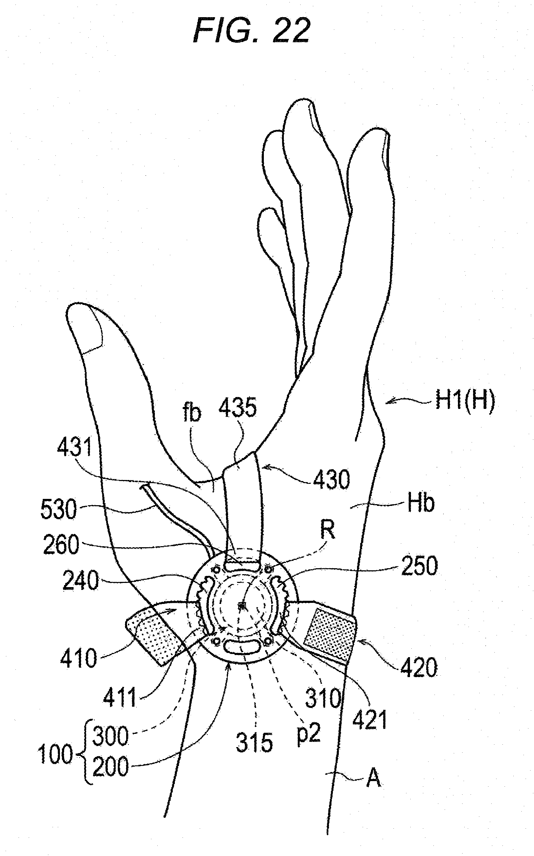

As illustrated in Figs. 16 and 22, the second puncture site p2 is a puncture site formed in the distal radial artery located on the distal side of the snuff box of the palmar artery running on the dorsal side Hb of the right hand H1 of the patient. The second puncture site p2 is located on the distal side of the right hand H1 with respect to the first puncture site p1 based on the extensor pollicis longus tendon t1 located in the dorsal side Hb of the right hand H1 of the patient.

Hereinafter, the hemostatic device 10 will be described in detail.

As schematically illustrated in Figs. 1, 2, 19, 20, and 21, the hemostatic device 10 includes a pressing member 100 configured to compress the first puncture site p1, a first band body 410 configured to be connectable to the pressing member 100, and a second band body 420 configured to be connectable to the pressing member 100.

As illustrated in Figs. 1, 2, 19, 20, and 21, the pressing member 100 includes a support member 200 and a pressing portion 300 fixed to the support member 200 and configured to compress the first puncture site p1.

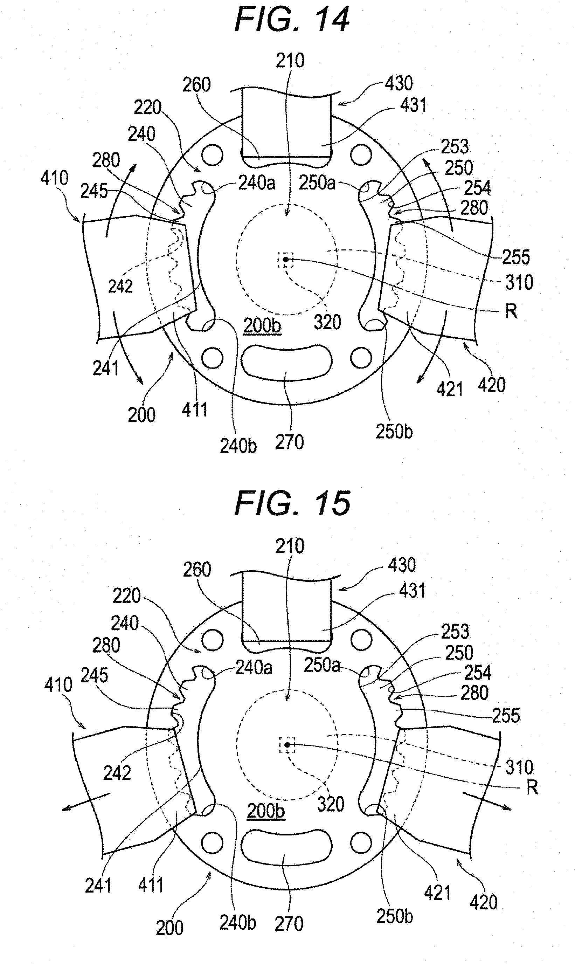

In the hemostatic device 10, as will be described later, angles of the first band body 410 and the second band body 420 in an extending direction with respect to the pressing portion 300 (angles in a radial direction centering around the pressing portion 300) can be changed by changing positions of the first band body 410 and the second band body 420 in a first hole portion 240 and a second hole portion 250 in a state where the first band body 410 and the second band body 420 are connected to the support member 200. The first band body 410 and the second band body 420 are configured to be able to slide and move along the first hole portion 240 and the second hole portion 250 centering around the pressing portion 300. Thus, the first band body 410 and the second band body 420 are configured to be able to slide and move along a predetermined direction based on a center point R located in a first region 210 of the support member 200 (see Fig. 14) .

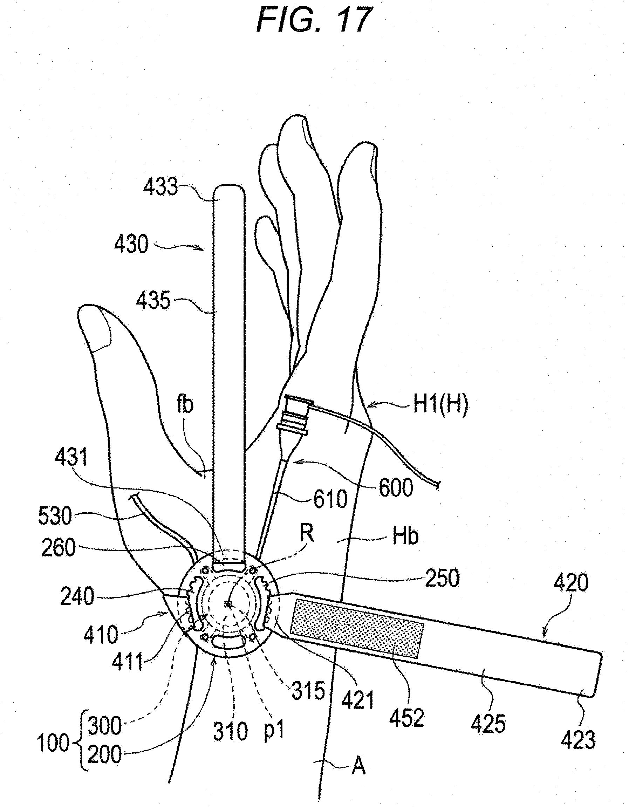

As illustrated in Figs. 17, 18, and 19, the first band body 410 and the second band body 420 can be disposed so as to be wrapped along an external periphery of the right hand H1 when the hemostatic device 10 is attached to the right hand H1 of the patient.

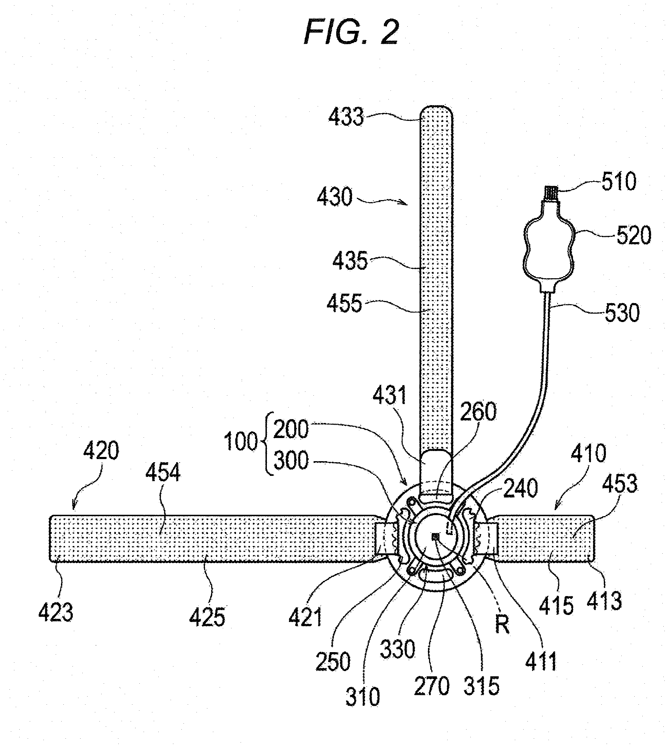

As illustrated in Figs. 1 and 2, a third band body 430 is connectable to the support member 200. As illustrated in Fig. 19, when the hemostatic device 10 is attached to the right hand H1 of the patient, the third band body 430 can be disposed so as to be hooked on an interdigital part fb located between two adjacent fingers (for example, the thumb and the index finger).

### <Pressing Portion>

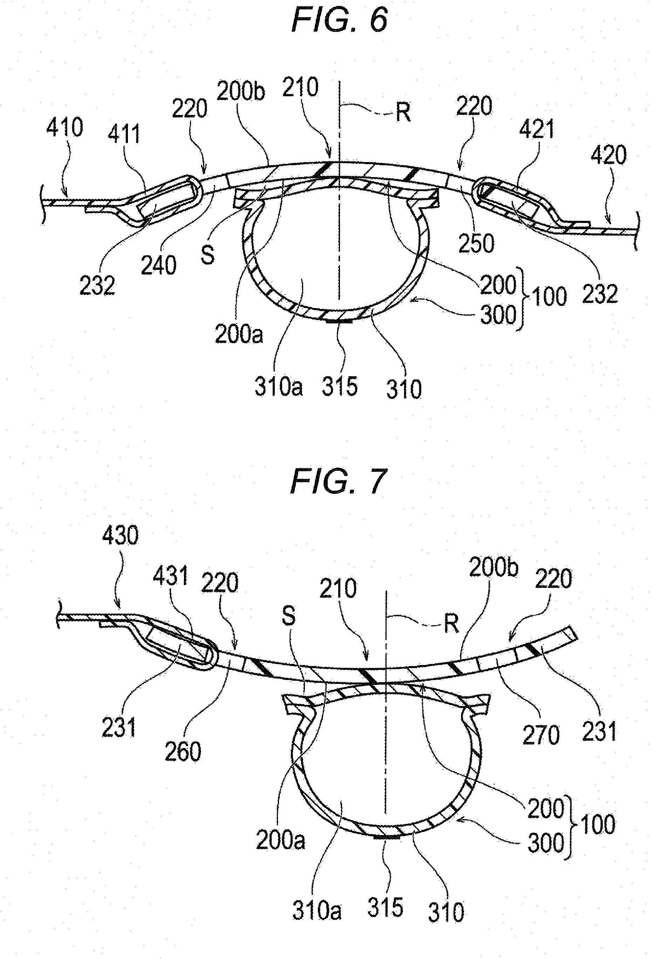

As illustrated in Figs. 6, 7, and 11, the pressing portion 300 can include an inflatable portion 310 that applies a compressive force to the first puncture site p1. In the present embodiment, a protruding portion 330 for fixing the inflatable portion 310 to the support member 200 is attached to the inflatable portion 310.

The inflatable portion 310 can be formed of, for example, a balloon made of a resin which allows inflow and discharge of a fluid such as air. Note that Figs. 6 and 7 are cross-sectional views illustrating a state in which the inflatable portion 310 inflates.

A tube 530 (see Figs. 1 and 2) to be described later is connected to a lumen 310a of the inflatable portion 310.

A material constituting the inflatable portion 310 is not particularly limited, and for example, polyvinyl chloride, polyethylene, polypropylene, polybutadiene, polyolefins such as ethylene-vinyl acetate copolymer (EVA), polyesters such as polyethylene terephthalate (PET) and polybutylene terephthalate (PBT), various thermoplastic elastomers such as polyvinylidene chloride, silicone, polyurethane, polyamide elastomer, polyurethane elastomer, polyester elastomer, nylon, nylon elastomer, or any combination thereof (such as a blend resin, a polymer alloy and a laminate) can be used.

As illustrated in Figs. 6, 7, 20, and 21, the inflatable portion 310 is fixed to one surface 200a of the support member 200. The one surface 200a of the support member 200 is a surface disposed on a body surface side of the hand H of the patient when the hemostatic device 10 is attached to the hand H of the patient. The other surface 200b of the support member 200 is a surface located on the opposite side of the one surface 200a.

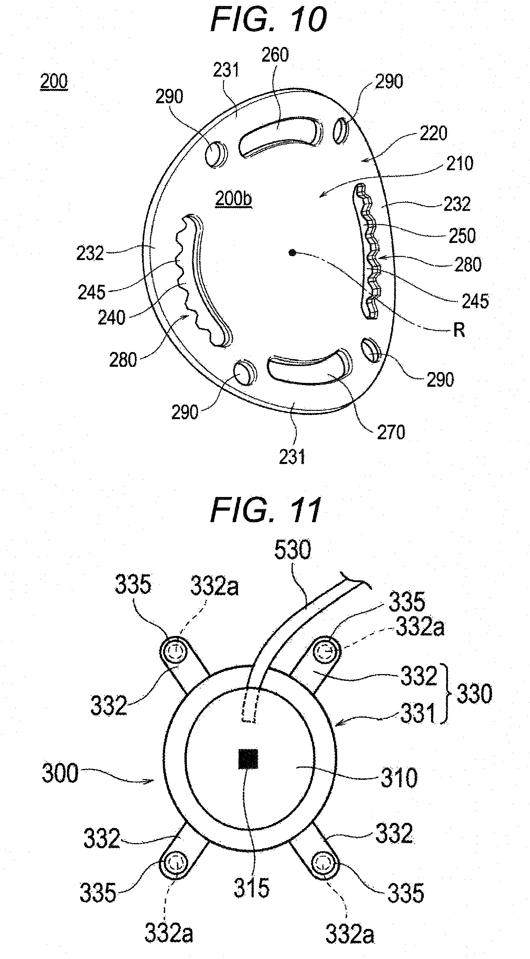

As illustrated in Fig. 11, the protruding portion 330 includes a flange portion 331 located around the inflatable portion 310 and a plurality of connection portions 332 protruding from the flange portion 331 in a predetermined direction.

The connection portions 332 protrude outward from an edge of the flange portion 331. Four connection portions 332 protruding in different directions are disposed on the protruding portion 330.

The inflatable portion 310 has a substantially circular shape in plan view illustrated in Figs. 1 to 5 and 11. The flange portion 331 has a substantially circular shape similarly to the inflatable portion 310. Each of the connection portions 332 has a structure protruding in a direction away from the flange portion 331 with a predetermined width.

Note that shapes of the inflatable portion 310 and the protruding portion 330 (the flange portion 331 and the connection portions 332) in plan view are not limited to the illustrated shapes. In addition, a cross-sectional shape before and after inflating of the inflatable portion 310, a constituent material of the inflatable portion 310, a specific structure of the inflatable portion 310, and the like, are also not particularly limited.

A fixing structure between the support member 200 and the pressing portion 300 (inflatable portion 310) will be described later.

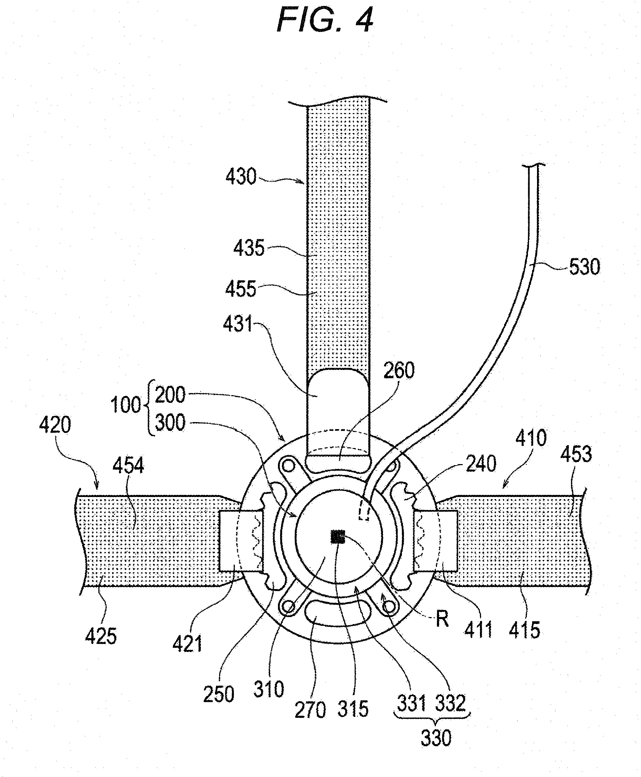

As illustrated in Figs. 4, 5, 6, 7, 20, and 21, a marker 315 for aligning the inflatable portion 310 with the first puncture site p1 is disposed in the inflatable portion 310.

The marker 315 is disposed on an outer surface of the inflatable portion 310 opposite to a surface on which the support member 200 is disposed (surface disposed on the body surface side of the hand H of the patient when the hemostatic device 10 is attached to the hand H of the patient).

A specific position of the marker 315 is not particularly limited as long as the inflatable portion 310 can be aligned with the puncture site. For example, the marker 315 may be disposed on an inner surface of the inflatable portion 310 opposite to a surface on which the support member 200 is disposed (surface disposed on the body surface side of the hand H of the patient when the hemostatic device 10 is attached to the hand H of the patient).

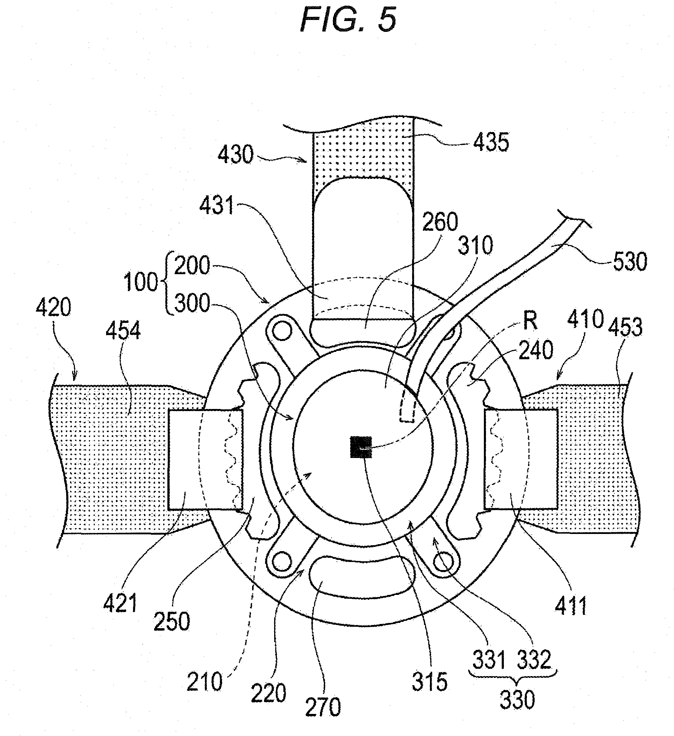

As illustrated in Fig. 5, the marker 315 is disposed at a substantially center position in a plane direction of the inflatable portion 310. The marker 315 is disposed so as to overlap with a substantially center position in a plane direction of the support member 200.

The marker 315 can be formed of, for example, a rectangular marker in which the entire marker 315 is formed in color. Note that a specific shape, a color, a forming method, a position, and the like, of the marker 315 are not particularly limited. For example, the marker 315 may include a transparent central portion and a colored linear frame portion surrounding the central portion. Further, for example, the marker 315 may be provided on the support member 200.

### <Support Member>

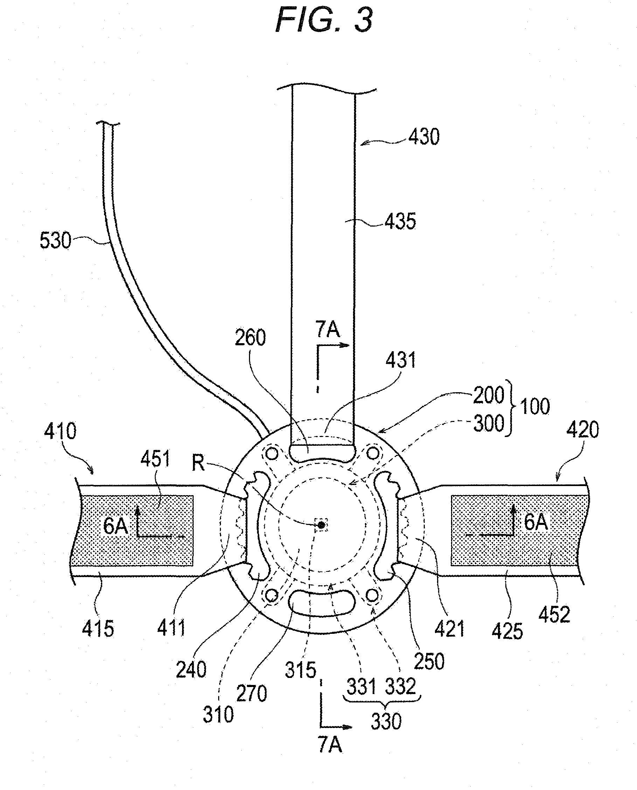

As illustrated in Figs. 3, 5, 6, and 7, the support member 200 includes a first region 210 in which the pressing portion 300 is disposed, and a second region 220 that is located outside the first region 210 and configured such that the first band body 410 and the second band body 420 are connectable.

The support member 200 has a circular shape in plan view illustrated in Fig. 5.

The first region 210 is a region where the pressing portion 300 overlaps in plan view illustrated in Fig. 5. The second region 220 is a region located outside the first region 210 in plan view illustrated in Fig. 5.

Note that the first region 210 can be arbitrarily defined based on an outer shape and a size of the pressing portion 300 disposed on the support member 200. In addition, the second region 220 can be defined based on a relative positional relationship with the first region 210. Thus, the first region 210 and the second region 220 can be appropriately changed according to the outer shape and the size of the pressing portion 300 disposed on the support member 200.

As illustrated in Figs. 5, 6, 7, and 15, a center point R, which is a center when the first band body 410 and the second band body 420 slide and move along the first hole portion 240 and the second hole portion 250, is located in the first region 210.

As illustrated in Figs. 5, 6, 7, and 15, the second region 220 includes the first hole portion 240 configured such that the first band body 410 is connectable, the second hole portion 250 located at a position facing the first hole portion 240 across the pressing portion 300 and configured such that the second band body 420 is connectable, a third hole portion 260 located at a position different from the hole portions 240 and 250 and configured such that the third band body 430 is connectable, and a fourth hole portion 270 located at a position facing the third hole portion 260 across the pressing portion 300.

The first hole portion 240, the second hole portion 250, the third hole portion 260, and the fourth hole portion 270 can be arranged in a circular shape along the outer shape of the support member 200 in plan view illustrated in Fig. 5. In the present embodiment, the support member 200 has a circular shape in plan view illustrated in Fig. 5. Thus, the hole portions 240, 250, 260, and 270 are arranged in a circular shape along a circumferential direction of the second region 220 located outside the first region 210 in the support member 200.

The above "arranged in a circular shape" means that assuming a case where a continuous hole is formed in the circumferential direction of the second region 220 of the support member 200, the hole portions 240, 250, 260, and 270 are arranged at intervals at arbitrary positions which are part of the hole.

When the hemostatic device 10 is attached to the right hand H1 of the patient, as illustrated in Fig. 19, the third hole portion 260 can be disposed on the distal side of the hand H with respect to the pressing portion 300, and the fourth hole portion 270 can be disposed on the proximal side of the hand H with respect to the pressing portion 300.

In a case where a position at which the hemostatic device 10 is attached to the hand H of the patient is changed, positions at which the third hole portion 260 and the fourth hole portion 270 are disposed with respect to the pressing portion 300 can be interchanged. In other words, the third hole portion 260 may be disposed on the proximal side of the hand H with respect to the pressing portion 300, and the fourth hole portion 270 may be disposed on the distal side of the hand H with respect to the inflatable portion 310. In a case where the fourth hole portion 270 is disposed on the distal side of the hand H with respect to the inflatable portion 310, the third band body 430 is connectable to the fourth hole portion 270, and the third band body 430 can be used for fixing the hemostatic device 10.

In the present embodiment, a width of a third one end portion 431 of the third band body 430 (width in a direction orthogonal to an extending direction of the third band body 430) is substantially the same as widths of the third hole portion 260 and the fourth hole portion 270. Thus, in a state where the third band body 430 is connected to the support member 200 via the third hole portion 260 or the fourth hole portion 270, sliding movement of the third band body 430 along each of the hole portions 260 and 270 is restricted.

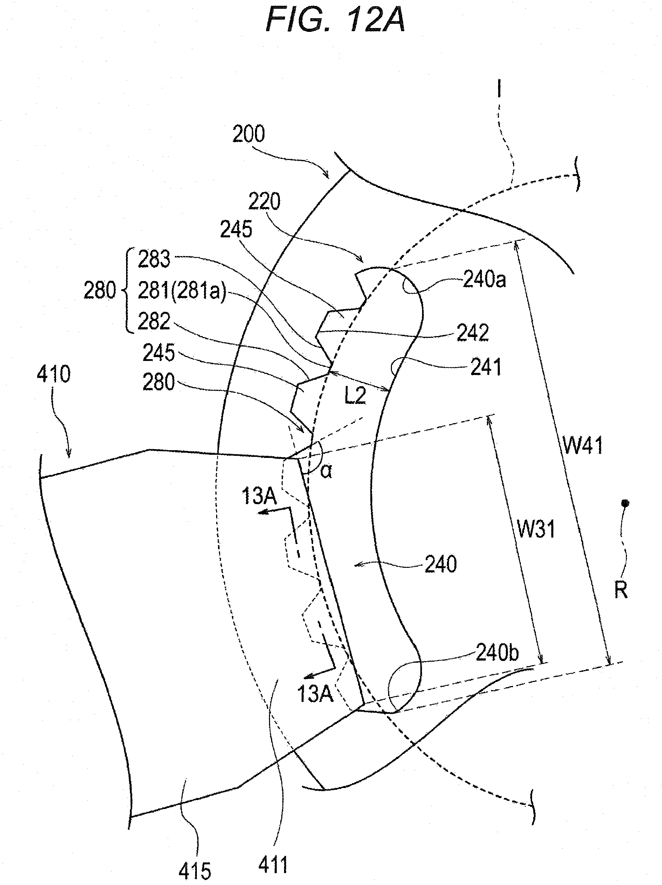

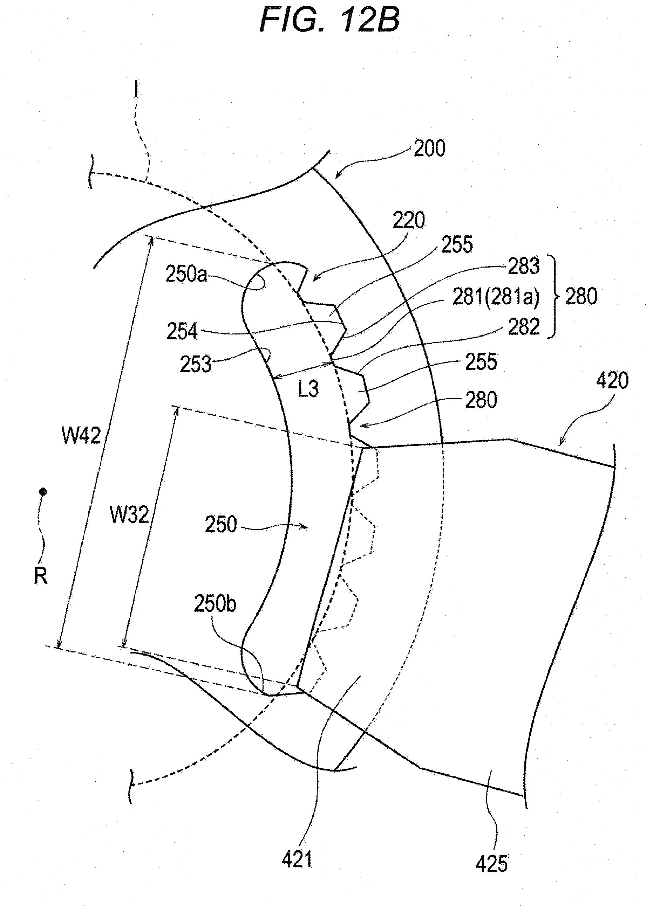

A width (see dimensions W41, W42 illustrated in Figs. 12A and 12B) of each of the hole portions 240, 250, 260, and 270 in the present specification means a linear distance between end portions 240a and 240b or between end portions 250a and 250b located in the extending direction of each of the hole portions 240, 250, 260, and 270.

Note that the support member 200 does not have to be provided with the third hole portion 260 and/or the fourth hole portion 270. In the hemostatic device 10, in a case where the third hole portion 260 and the fourth hole portion 270 are not provided in the support member 200, use of the third band body 430 can be omitted.

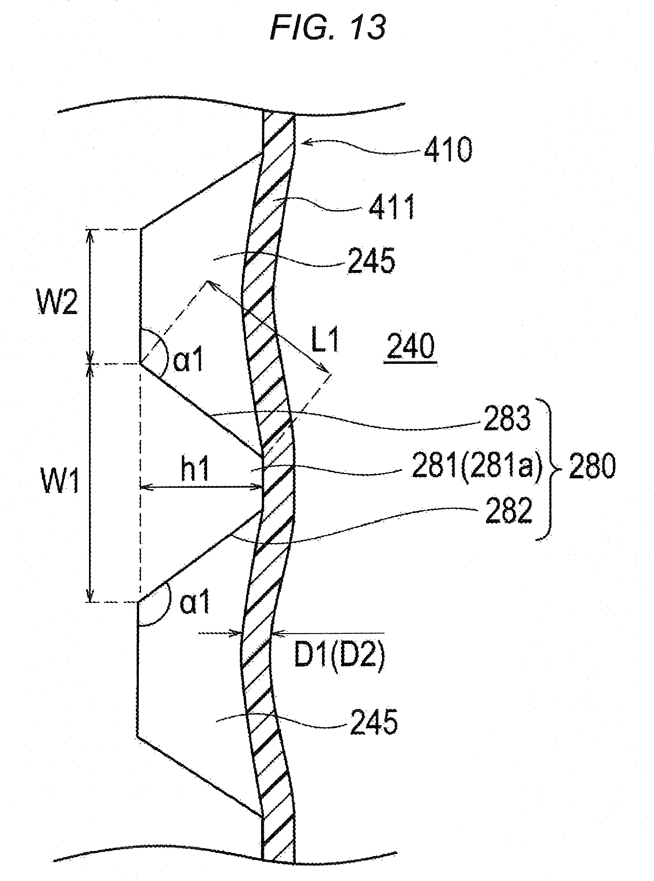

As illustrated in Fig. 12A, the width W41 of the first hole portion 240 is larger than the width W31 of a first one end portion 411 of the first band body 410 (width in a direction orthogonal to an extending direction of the first band body 410). As illustrated in Figs. 12A and 13, a distance L2 between a protrusion 280 located in the first hole portion 240 and a first side surface 241 is larger than a thickness D1 of the first one end portion 411 of the first band body 410. Thus, the first band body 410 can slide and move between the one end portion 240a of the first hole portion 240 and the other end portion 240b of the first hole portion 240 as indicated by an arrow in Fig. 14 in a state where the first band body 410 is connected to the first hole portion 240.

Note that in the present embodiment, a width W31 of the first one end portion 411 of the first band body 410 is formed to be smaller than a width of a first body portion 415. For example, in a case where a width of the first band body 410 is formed to be substantially constant along the extending direction of the first band body 410, the width W31 of the first one end portion 411 is the same as the width of the first band body 410. The same applies to the second band body 420.

As illustrated in Fig. 12B, a width W42 of the second hole portion 250 is larger than a width W32 of a second one end portion 421 of the second band body 420 (width in a direction orthogonal to an extending direction of the second band body 420). As illustrated in Figs. 12B and 13, a distance L3 between the protrusions 280 located in the second hole portion 250 and a second side surface 242 is larger than a thickness D2 of the second one end portion 421 of the second band body 420. Thus, the second band body 420 can slide and move between one end portion 250a of the second hole portion 250 and the other end portion 250b of the second hole portion 250 as indicated by an arrow in Fig. 14 in a state where the second band body 420 is connected to the second hole portion 250.

Ranges in which the first band body 410 and the second band body 420 can slide and move are not particularly limited, but for example, each of the band bodies 410 and 420 can be configured to be able to slide and move in a range from 1° to 75° along the first hole portion 240 and the second hole portion 250 based on the center point R.

Note that in order to make sliding movement of each of the band bodies 410 and 420 more reliable, the distance L2 between the protrusions 280 located in the first hole portion 240 and the first side surface 241 and the distance L3 between the protrusions 280 located in the second hole portion 250 and the third side surface 253 (see Figs. 12A and 12B) are widened toward the inflatable portion 310 side, the inflatable portion 310 and the band bodies 410 and 420 easily interfere with each other when the band bodies 410 and 420 slide and move. On the other hand, if the distances L2 and L3 are widened to a peripheral edge side of the support member 200, an area of a material constituting the support member 200 decreases, so that strength of the support member 200 may decrease and the compressive force on the first puncture site p1 may decrease. In consideration of the above points, each of the distances L2 and L3 can be formed so as not to have excessively large dimensions within a range that enables sliding movement of each of the band bodies 410 and 420.

As illustrated in Figs. 12A and 12B, the first hole portion 240 and the second hole portion 250 have a plurality of protrusions 280 protruding toward the pressing portion 300 (side on which the center point R is located in Figs. 12A and 12B).

As illustrated in Fig. 12A, the first hole portion 240 has the first side surface 241 and the second side surface 242 which is located on a side farther away from the pressing portion 300 than the first side surface 241 and which includes a plurality of protrusions 280.

The second side surface 242 includes gap portions 245 formed between adjacent protrusions 280 among the plurality of protrusions 280. On the second side surface 242, the protrusions 280 and the gap portions 245 are alternately arranged along the extending direction of the first hole portion 240. In the first hole portion 240, the protrusions 280 and the gap portions 245 are disposed over the entire second side surface 242 in the extending direction.

As illustrated in Fig. 12B, the second hole portion 250 has the third side surface 253 and the fourth side surface 254 which is located on a side farther away from the pressing portion 300 than the third side surface 253 and which includes a plurality of protrusions 280.

The fourth side surface 254 includes gap portions 255 formed between adjacent protrusions 280 among the plurality of protrusions 280. On the fourth side surface 254, the protrusions 280 and the gap portions 255 are alternately arranged along the extending direction of the second hole portion 250. In the second hole portion 250, the protrusions 280 and the gap portions 255 are disposed over the entire fourth side surface 254 in the extending direction.

As illustrated in Figs. 12A and 12B, no protrusion 280 is formed on the first side surface 241 and the third side surface 253. The first side surface 241 and the third side surface 253 are flat surfaces curved in a concave shape toward a side away from the inflatable portion 310 of the pressing portion 300.

As illustrated in Figs. 12A and 13, each of the protrusions 280 formed on the second side surface 242 and the fourth side surface 254 includes a central portion 281 including an apex of the protrusion 280 and a pair of connection portions 282 and 283 connecting the central portion 281 and the gap portions 245 and 255.

The connection portions 282 and 283 tilt from the central portion 281 toward the gap portions 245 and 255. The connection portions 282 and 283 extend substantially linearly from the central portion 281 toward the gap portions 245 and 255.

As illustrated in Figs. 12A and 12B, at least part of the central portion 281 of each of the plurality of protrusions 280 located in the first hole portion 240 and at least part of the central portion 281 of each of the plurality of protrusions 280 located in the second hole portion 250 are located on the same virtual circle. A broken line I indicates part of the virtual circle connecting the central portions 281 of the protrusions 280 in plan view. In the present embodiment, a center position of the virtual circle indicated by the broken line I overlaps with the center point R located substantially at the center of the support member 400.

As illustrated in Figs. 12A and 12B, the protrusion 280 can be formed in a trapezoidal shape in plan view. In a case where the protrusion 280 has a trapezoidal shape, the central portion 281 of the protrusion 280 can include a linear flat portion 281a. In the present embodiment, the entire central portion 281 is formed by the flat portion 281a. However, the flat portion 281a may be provided only in part of the central portion 281.

As illustrated in Fig. 12A, an attachment angle α formed between the first one end portion 411 of the first band body 410 pressed against the protrusions 280 and each of the connection portions 282 and 283 is preferably 90° or more and 135° or less. In a case where the attachment angle α is formed at an acute angle, it is possible to enhance a locking function when each of the band bodies 410 and 420 is pressed against the protrusions 280. However, there is a possibility that smooth sliding movement of the band bodies 410 and 420 is hindered. From such a viewpoint, the attachment angle α is preferably 90° or more and 135° or less.

Note that in a case where the attachment angle α is 90° or more and 135° or less, an inclination angle α1 (see Fig. 13) of the protrusion 280 is preferably 110° or more and 155° or less.

Note that the protrusion 280 can also be formed in, for example, a triangle having an attachment angle α of 135° or less.

Fig. 13 schematically illustrates a cross section of the first one end portion 411 and the first hole portion 240 of the first band body 410 along an arrow 13A-13A illustrated in Fig. 12A. A magnitude relationship between portions described below will be mainly described by taking the first band body 410 and the first hole portion 240 as an example. Note that, in the hemostatic device 10, a magnitude relationship of dimensions similar to those of the first band body 410 and the first hole portion 240 can be adopted between the second band body 420 and the second hole portion 250.

As illustrated in Fig. 13, the length L1 of the connection portions 282 and 283 can be formed to be longer than the thickness D1 of the first band body 410 and the thickness D2 of the second band body 420. In the present embodiment, the length L1 of each of the connection portions 282 and 283 is a length of a linear component of the length L1 of each of the connection portions 282 and 283. The thickness D1 of the first band body 410 and the thickness D2 of the second band body 420 are thicknesses in a natural state in which no tensile force, or the like, is applied to the band bodies 410 and 420.

In the hemostatic device 10, the length L1 of the connection portions 282 and 283 is formed to be longer than the thickness D1 of the first band body 410 and the thickness D2 of the second band body 420 as described above, and thus, when each of the band bodies 410 and 420 is pressed against the protrusions 280, part of each of the band bodies 410 and 420 can be pushed into the gap portions 245 and 255 as illustrated in Fig. 13.

As illustrated in Fig. 13, the width W2 of the gap portions 245 and 255 can be formed smaller than the width W1 of the protrusion 280. The widths of the gap portions 245 and 255 are lengths along the extending direction of the hole portions 240 and 250. The width W1 of the protrusion 280 is a length along the extending direction of each of the hole portions 240 and 250 and is a portion having the largest length in the protrusion 280. In the present embodiment, the width of the protrusion 280 is the width of a bottom portion of the trapezoid.

In the hemostatic device 10, the width W2 of the gap portions 245 and 255 is smaller than the width W1 of the protrusion 280 as described above, so that it is possible to prevent most of each of the band bodies 410 and 420 from entering the gap portions 245 and 255 in a state where each of the band bodies 410 and 420 is connected to each of the hole portions 240 and 250.

As illustrated in Figs. 12A, 12B, and 13, the width W1 of the protrusion 280 can be formed to be smaller than the width W31 of the first one end portion 411 of the first band body 410 and the width W32 of the second one end portion 421 of the second band body 420.

As described above, in the hemostatic device 10, the width W1 of the protrusion 280 is smaller than the width W31 of the first one end portion 411 of the first band body 410 and the width W32 of the second one end portion 421 of the second band body 420, so that it is possible to push part of each of the one end portions 411 and 421 of the band bodies 410 and 420 into each of the gap portions 245 and 255 when each of the band bodies 410 and 420 are pressed against the protrusions 280.

As illustrated in Figs. 12A, 12B, and 13, a height h1 of the protrusion 280 from the first hole portion 240 side and the second hole portion 250 side toward the pressing portion 300 side can be formed to be larger than the thickness D1 of the first band body 410 and the thickness D2 of the second band body 420. In the present embodiment, the thickness D1 of the first band body 410 is the same as the thickness D2 of the second band body 420.

In the hemostatic device 10, the height h1 of the protrusion 280 is larger than the thickness D1 of the first band body 410 and the thickness D2 of the second band body 420 as described above, and thus, when each of the band bodies 410 and 420 is pressed against the protrusions 280, part of each of the band bodies 410 and 420 can be pushed into the gap portions 245 and 255 as illustrated in Fig. 13.

As illustrated in Fig. 13, the width W2 of each of the gap portions 245 and 255 can be formed to be smaller than the width W31 of the first one end portion 411 of the first band body 410 and the width W32 of the second one end portion 421 of the second band body 420.

In the hemostatic device 10, the width W2 of each of the gap portions 245 and 255 is smaller than the width W31 of the first one end portion 411 of the first band body 410 and the width W32 of the second one end portion 421 of the second band body 420 as described above, so that it is possible to prevent the entire width direction of each of the one end portions 411 and 421 of each of the band bodies 410 and 420 from entering into each of the gap portions 245 and 255 when each of the band bodies 410 and 420 is pressed against the protrusions 280.

Functions of the protrusions 280 will be described with reference to Figs. 14 and 15.

As illustrated in Fig. 14, in the hemostatic device 10, in a state where the first band body 410 is not pressed against the second side surface 242 on which the protrusions 280 are formed, the first band body 410 can slide and move along the extending direction of the first hole portion 240. Similarly, in the hemostatic device 10, in a state where the second band body 420 is not pressed against the fourth side surface 254 on which the protrusions 280 are formed, the second band body 420 can slide and move along the extending direction of the second hole portion 250. Each of the band bodies 410 and 420 can slide and move when the hemostatic device 10 is attached to the right hand H1 of the patient, and thus, a degree of freedom of a position at which each of the band bodies 410 and 420 is attached to the right hand H1 is high.

As illustrated in Fig. 15, in the hemostatic device 10, when each of the band bodies 410 and 420 is pulled toward each of the side surfaces 242 and 254 and pressed against the protrusions 280 in a state where the hemostatic device 10 is attached to the right hand H1 of the patient, the protrusions 280 bite into each of the band bodies 410 and 420. In the hemostatic device 10, if the protrusions 280 bite into each of the band bodies 410 and 420, at least one of both end portions located in the width direction of each of the band bodies 410 and 420 fits into the gap portions 245 and 255. The protrusion 280 closest to the end portion fitted in the gap portions 245 and 255 serves as a stopper for restricting sliding movement of each of the band bodies 410 and 420. As a result, the hemostatic device 10 can effectively exhibit the locking function by the protrusions 280.

In a case where the pressing portion 300 is configured by the inflatable portion 310 that can inflate, the operator can apply a tensile force toward each of the side surfaces 242 and 254 to each of the band bodies 410 and 420 by inflating the inflatable portion 310. The hemostatic device 10 can cause the protrusions 280 to bite into each of the band bodies 410 and 420 accompanying inflating of the inflatable portion 310 by applying the above-described tensile force to each of the band bodies 410 and 420 and pressing each of the band bodies 410 and 420 against each of the side surfaces 242 and 254. In the hemostatic device 10, if the inflatable portion 310 deflates, a state where the protrusions 280 bite into each of the band bodies 410 and 420
is resolved. Thus, the operator can restrict and release the restriction of sliding movement of each of the band bodies 410 and 420 by operating inflating and deflating of the inflatable portion 310.

Note that the operator can also restrict the sliding movement of each of the band bodies 410 and 420 by wrapping and fixing each of the band bodies 410 and 420 around the right hand H1 of the patient while causing the protrusions 280 to bite into each of the band bodies 410 and 420 in a state where each of the band bodies 410 and 420 is pulled toward each of the side surfaces 242 and 254.

The hemostatic device 10 has the gap portions 245 and 255 formed between the adjacent protrusions 280 (see Figs. 12A and 12B). In the hemostatic device 10, when each of the band bodies 410 and 420 is pressed against the protrusions 280, portions adjacent to portions in contact with the protrusions 280 in each of the band bodies 410 and 420 can be pushed into the gap portions 245 and 255. Thus, the hemostatic device 10 can cause the protrusions 280 to firmly bite into each of the band bodies 410 and 420.

The connection portions 282 and 283 of the protrusion 280 tilt from the central portion 281 of the protrusion 280 toward the gap portions 245 and 255. The connection portions 282 and 283 tilt, and thus, when each of the band bodies 410 and 420 are pressed against the protrusions 280, portions adjacent to the portions in contact with the protrusions 280 in each of the band bodies 410 and 420 are easily guided along the connection portions 282 and 283 and pushed into the gap portions 245 and 255. Thus, the hemostatic device 10 can cause the protrusions 280 to bite into each of the band bodies 410 and 420 more firmly.

In the hemostatic device 10, at least part of the central portion 281 of each of the plurality of protrusions 280 located in the first hole portion 240 and at least part of the central portion 281 of each of the plurality of protrusions 280 located in the second hole portion 250 are located on the same virtual circle I. In other words, the hemostatic device 10 has no variation in the position in a height direction (protruding direction) of the protrusions 280. Thus, in the hemostatic device 10, it is possible to prevent each of the band bodies 410 and 420 from being caught by the protrusions 280 in a state where the protrusions 280 do not bite into each of the band bodies 410 and 420. By this means, in the hemostatic device 10, each of the band bodies 410 and 420 can smoothly slide and move.

In the hemostatic device 10, the protrusion 280 has a trapezoidal shape. The central portion 281 of the protrusion 280 includes a linear flat portion 281a. The hemostatic device 10 is configured such that the central portion 281 includes the flat portion 281a, so that it is possible to prevent each of the band bodies 410 and 420 from being caught by the central portion 281 when each of the band bodies 410 and 420 slides and moves. Thus, each of the band bodies 410 and 420 can smoothly slide and move.

In the hemostatic device 10, as illustrated in Figs. 12A and 12B, the protrusions 280 and the gap portions 245 and 255 are disposed on the entire side surfaces 242 and 254 of the hole portions 240 and 250 in the extending direction of the hole portions 240 and 250. Thus, even in a case where each of the band bodies 410 and 420 is disposed at any position in the extending direction of each of the hole portions 240 and 250, the protrusions 280 can be caused to bite into each of the band bodies 410 and 420 by pressing each of the band bodies 410 and 420 against each of the side surfaces 242 and 254.

As illustrated in Figs. 7, 8, 9, 10, and 21, in portions where the third hole portion 260 and the fourth hole portion 270 are disposed in the second region 220 of the support member 200, a first curved region 231 curved in a convex shape toward a side away from the inflatable portion 310 disposed on one surface 200a of the support member 200 is formed.

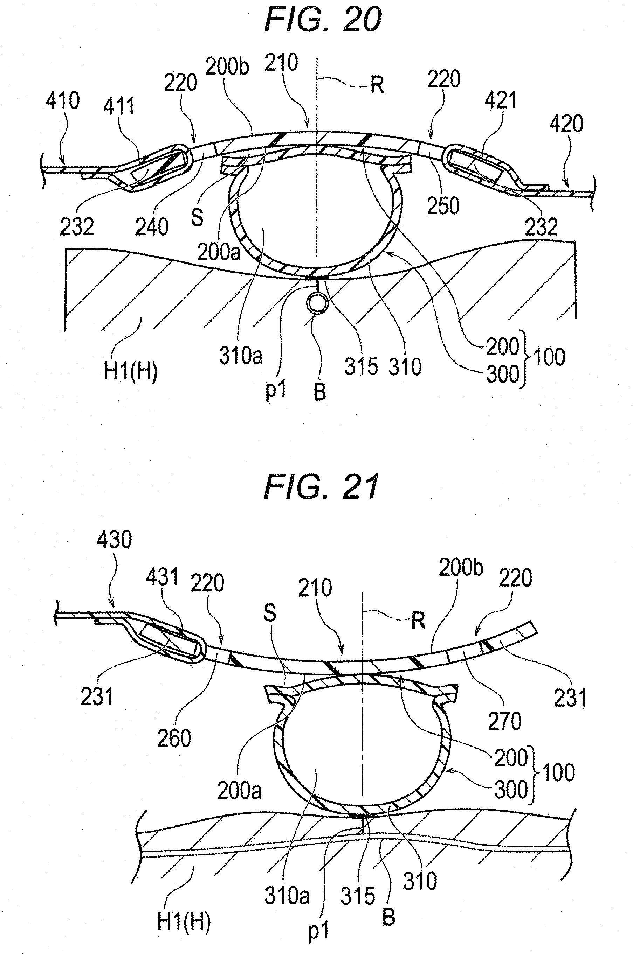

The above-described "side away from the inflatable portion 310" is a side away from the body surface of the hand H of the patient (upper side in Fig. 21) when the hemostatic device 10 is attached to the hand H of the patient.

As illustrated in Figs. 6, 8, 9, and 10, in portions where the first hole portion 240 and the second hole portion 250 are disposed in the second region 220 of the support member 200, a second curved region 232 curved in a convex shape toward the inflatable portion 310 side disposed on the one surface 200a of the support member 200 is formed.

The above-described "inflatable portion 310 side" is a side (lower side in Fig. 20) close to the body surface of the hand H of the patient when the hemostatic device 10 is attached to the hand H of the patient.

The support member 200 is preferably made of a material having predetermined hardness. In a case where the support member 200 is configured to have predetermined hardness, as illustrated in Figs. 20 and 21, when the inflatable portion 310 applies a compressive force to the first puncture site p1 formed on the right hand H1 of the patient, the support member 200 can press the inflatable portion 310 against the right hand H1 of the patient. Accordingly, it is possible to prevent the inflatable portion 310 from floating from the right hand H1 of the patient.

As a constituent material of the support member 200 having hardness as described above, for example, acrylic resin, polyvinyl chloride (particularly hard polyvinyl chloride), polyolefin such as polyethylene, polypropylene, or polybutadiene, polystyrene, poly- (4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, polyethylene terephthalate (PET), or the like, can be used.

In each of the inflatable portion 310 and the support member 200, portions overlapping each other in plan view illustrated in Figs. 4 and 5 can be formed transparent. In a case where the inflatable portion 310 and the support member 200 are configured in this manner, as illustrated in Figs. 20 and 21, when the hemostatic device 10 is attached to the right hand H1 of the patient, the operator can visually confirm the position of the marker 315 and/or the first puncture site p1 through the inflatable portion 310 and the support member 200 more easily. Note that the term "transparent" includes colored transparent, colorless transparent, and translucent.

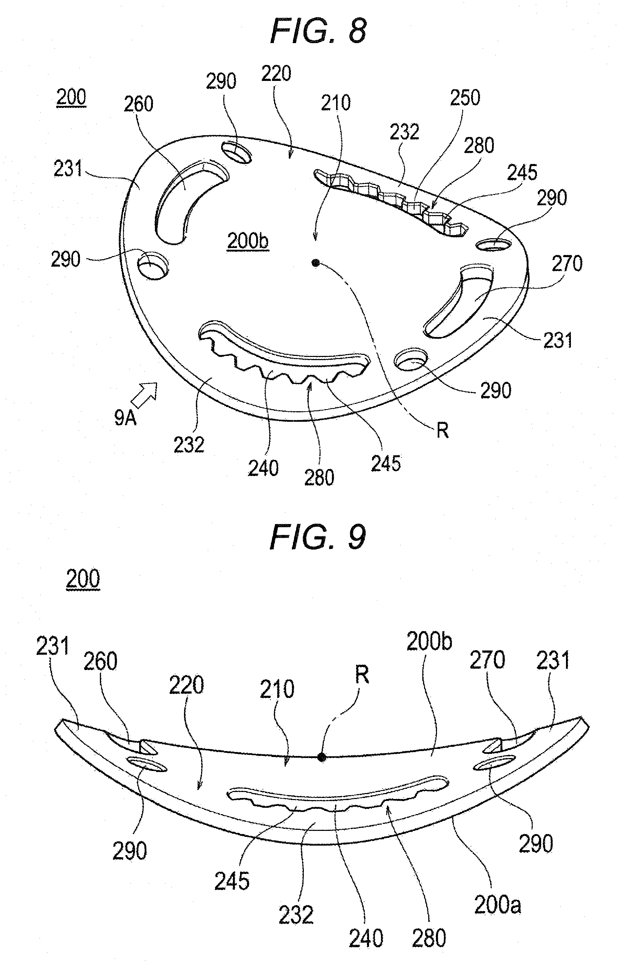

As illustrated in Figs. 8, 9, and 10, the support member 200 has an attachment hole 290 formed in the second region 220. For example, two attachment holes 290 can be provided at predetermined positions near the third hole portion 260 located in the first curved region 231. Further, for example, the two attachment holes 290 can be provided at predetermined positions near the fourth hole portion 270 located in the first curved region 231.

As illustrated in Fig. 11, each connection portion 332 of the protruding portion 330 has a through-hole 332a.

The pressing portion 300 can be fixed to the support member 200 by a fixing member 335 (see Fig. 11) to be inserted into the through-hole 332a of the connection portion 332 and the attachment hole 290 of the support member 200. The fixing member 335 can be formed of, for example, a caulking pin caulked to the through-hole 332a and the attachment hole 290. Note that illustration of the fixing member 335 is omitted in the drawings other than Fig. 11.

As described above, the first curved region 231 and the second curved region 232 of the support member 200 are curved in a predetermined direction. The inflatable portion 310 (pressing portion 300) is fixed to the support member 200 at four points via four through-holes 332a respectively formed in the four connection portions 332. In other words, the inflatable portion 310 is not fixed in a state where the entire surface of the inflatable portion 310 facing the support member 200 is in contact with the one surface 200a of the support member 200. Thus, as illustrated in Figs. 6, 7, 20, and 21, in a state where the inflatable portion 310 inflates, the inflatable portion 310 forms a predetermined space S between an outer peripheral portion of the inflatable portion 310 in plan view and the support member 200.

For example, if the entire surface of the inflatable portion 310 facing the support member 200 is fixed to the support member 200 with an adhesive, or the like, when the inflatable portion 310 inflates, the inflatable portion 310 may also inflate in a lateral direction with respect to the direction toward the first puncture site p1. As a result, the compressive force on the first puncture site p1 by the inflatable portion 310 is dispersed, and the compressive force cannot be effectively applied to the first puncture site p1. On the other hand, in the hemostatic device 10, the inflatable portion 310 and the support member 200 are fixed such that the space S is formed between the inflatable portion 310 and each of the curved regions 231 and 232 of the support member 200. Thus, when the inflatable portion 310 inflates, it is possible to suppress the inflatable portion 310 from inflating in the lateral direction with respect to the direction toward the first puncture site p1. As a result, in the hemostatic device 10, it is possible to prevent the compressive force on the first puncture site p1 by the inflatable portion 310 from being dispersed and effectively apply the compressive force to the first puncture site p1.

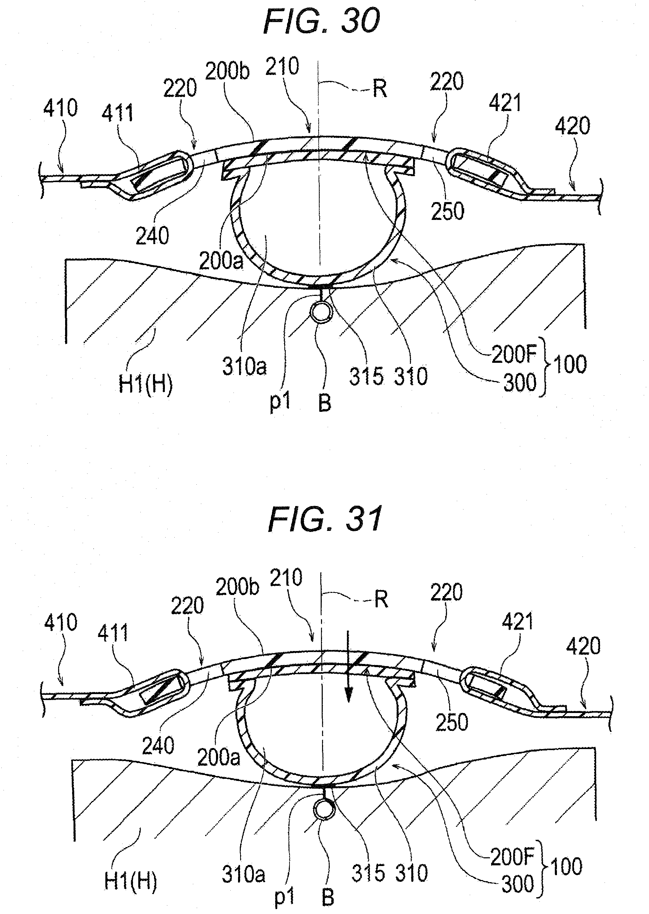

Note that a method for fixing the inflatable portion 310 to the support member 200 is not limited to the method using the attachment hole 290 of the support member 200 and the through-hole 332a of the protruding portion 330. For example, as described in the later-described modification (see Figs. 30 and 31), part or the whole of the surface of the inflatable portion 310 located on the support member 200 side may be fixed to the one surface 200a of the support member 200 with an adhesive, or the inflatable portion 310 may be fixed to the support member 200 via another connection member (for example, a sheet-like member) disposed between the inflatable portion 310 and the support member 200.

### <Band Body>

As illustrated in Figs. 1, 2, 3, and 4, the first band body 410 includes a first body portion 415, a first one end portion 411 configured to be connectable to the first hole portion 240 of the support member 200, and a first free other end portion 413.

As illustrated in Figs. 1 and 2, the first body portion 415 extends along a longitudinal direction of the first band body 410.

Note that the "free other end portion" in the present specification means that there is no direct or indirect connection relationship with other members in a state where the hemostatic device 10 is not attached (state where the hemostatic device is not attached to the patient).

As illustrated in Fig. 6, the first one end portion 411 of the first band body 410 can be disposed so as to be inserted into and wrapped around the first hole portion 240 of the support member 200. The first band body 410 is connectable to the support member 200 via the first one end portion 411 and the first hole portion 240.

As illustrated in Figs. 1, 2, 3, and 4, the second band body 420 includes a second body portion 425, a second one end portion 421 configured to be connectable to the second hole portion 250 of the support member 200, and a second free other end portion 423.

As illustrated in Figs. 1 and 2, the second body portion 425 extends along a longitudinal direction of the second band body 420.

As illustrated in Fig. 6, the second one end portion 421 of the second band body 420 can be disposed so as to be inserted into and wrapped around the second hole portion 250 of the support member 200. The second band body 420 is connected to the support member 200 via the second one end portion 421 and the second hole portion 250.

As illustrated in Figs. 1, 2, 3, and 4, the third band body 430 includes a third body portion 435, a third one end portion 431 configured to be connectable to the third hole portion 260 of the support member 200, and a third free other end portion 433.

As illustrated in Figs. 1 and 2, the third body portion 435 extends along a longitudinal direction of the third band body 430.

As illustrated in Fig. 7, the third one end portion 431 of the third band body 430 can be disposed so as to be inserted into and wrapped around the third hole portion 260 of the support member 200. The third band body 430 is connected to the support member 200 via the third one end portion 431 and the third hole portion 260.

Note that a structure of connecting the band bodies 410, 420, and 430 to the hole portions 240, 250, and 260 is not particularly limited. For example, a fixing member (for example, a hook-and-loop fastener) capable of holding and releasing a state where each of the one end portions 411, 421, and 431 is wrapped around each of the hole portions 240, 250, and 260 can be disposed at each of the one end portions 411, 421, and 431 of each of the band bodies 410, 420, and 430. In a case where each of the band bodies 410, 420, and 430 is configured in this manner, each of the band bodies 410, 420, and 430 has a structure that is connectable to and separated from the support member 200.

A constituent material of each of the band bodies 410, 420, and 430 is not particularly limited, and can be made of, for example, a vinyl chloride resin, a polyamide resin, a polyamide elastomer resin, a polyurethane resin, a polyester resin, or the like. In addition, a specific shape (shape in plan view or cross-sectional shape), length, and the like, of each of the band bodies 410, 420, and 430 are not particularly limited.

### <Fixing Portion>

As illustrated in Figs. 1, 2, 3, and 4, the hemostatic device 10 can be configured to include five fixing portions of a first fixing portion 451, a second fixing portion 452, a third fixing portion 453, a fourth fixing portion 454, and a fifth fixing portion 455.

As illustrated in Figs. 1 and 3, the first fixing portion 451 is disposed on the outer surface of the first band body 410. The second fixing portion 452 is disposed on the outer surface of the second band body 420.

As illustrated in Figs. 2 and 4, the third fixing portion 453 is disposed on the inner surface of the first band body 410. The fourth fixing portion 454 is disposed on the inner surface of the second band body 420. The fifth fixing portion 455 is disposed on the inner surface of the third band body 430. Note that the third fixing portion 453 disposed on the first band body 410 is used when the first band body 410 is fixed to the support member 200. Thus, the third fixing portion 453 disposed on the first band body 410 is not used for the purpose of fixing the hemostatic device 10 to the forearm A of the patient.

The inner surface of each of the band bodies 410, 420, and 430 is a surface disposed on the body surface side of the patient when the hemostatic device 10 is attached to the patient, and the outer surface of each of the band bodies 410, 420, and 430 is a surface located on the opposite side to the inner surface.

The first fixing portion 451 and the second fixing portion 452 are constituted as a male side of the hook-and-loop fastener. The third fixing portion 453, the fourth fixing portion 454, and the fifth fixing portion 455 are constituted as a female side of the hook-and-loop fastener. The hook-and-loop fastener in the present specification is a hook-and-loop fastener, and is, for example, Magic Tape (registered trademark) or Velcro (registered trademark).

Note that a specific structure of each of the fixing portions 451, 452, 453, 454, and 455 is not limited as long as it is possible to fix the inflatable portion 310 to the right hand H1 of the patient by connecting the band bodies 410, 420, and 430 to each other in a state where the hemostatic device 10 is disposed on the right hand H1 of the patient. For example, it is possible to arbitrarily omit installation of some fixing portions and change positions at which the fixing portions are disposed in each of the band bodies 410, 420, and 430. In a case where each of the fixing portions 451, 452, 453, 454, and 455 is formed of a hook-and-loop fastener, the male side and the female side of the hook-and-loop fastener may be interchanged. Further, each of the fixing portions 451, 452, 453, 454, and 455 may be, for example, a snap, a button, a clip, a frame member in which a hole portion is formed, or the like.

### <Injection Portion>

As illustrated in Figs. 1 and 2, the hemostatic device 10 has an injection portion 510 for injecting a fluid into the inflatable portion 310.

The injection portion 510 is formed of a connector incorporating a check valve (not illustrated). A syringe (not shown) may be connected to the injection portion 510.

A buffer member 520 having an inflatable space is disposed between the injection portion 510 and the inflatable portion 310. The buffer member 520 is formed of a flexible bag-shaped member in which a space is formed. Note that the buffer member 520 may be provided with an arrow-shaped marker indicating an insertion direction of the syringe into the injection portion 510.

The injection portion 510 is connected to one end side of the buffer member 520. A lumen of the injection portion 510 communicates with the space of the buffer member 520. However, while the check valve incorporated in the injection portion 510 is closed, communication between the lumen of the injection portion 510 and the space of the buffer member 520 is blocked.

A tube 530 having flexibility is connected to the other end side of the buffer member 520. The lumen of the tube 530 communicates with the space of the buffer member 520. Further, the other end portion of the tube 530, opposite to the one end portion connected to the buffer member 520 is connected to the inflatable portion 310. The lumen of the tube 530 communicates with the lumen 310a of the inflatable portion 310.

To inflate the inflatable portion 310, the operator inserts a cylindrical distal end portion of the syringe (not illustrated) into the injection portion 510 and opens the check valve. The operator injects air in the syringe into the lumen 310a of the inflatable portion 310 by pushing a pusher of the syringe in a state where the check valve of the injection portion 510 is opened.

When air is injected into the lumen 310a of the inflatable portion 310, the inflatable portion 310 inflates. When the inflatable portion 310 inflates, the buffer member 520 communicating with the lumen 310a of the inflatable portion 310 via the tube 530 inflates. By visually confirming inflating of the buffer member 520, the operator can easily grasp that the inflatable portion 310 inflates without leakage of air.

To deflate the inflatable portion 310, the operator inserts the cylindrical distal end portion of the syringe into the injection portion 510 and pulls the pusher of the syringe. The operator can discharge the air in the lumen 310a of the inflatable portion 310 to the syringe by performing the above operation.

### <Use Examples of Hemostatic Device>

Next, a first use example of the hemostatic device 10 will be described with reference to Figs. 17 to 21.

In the first use example, procedure of using the hemostatic device 10 to stop bleeding at the first puncture site p1 formed on the right hand H1 of the patient will be described.

Fig. 17 illustrates a state in which the sheath tube 610 of the introducer 600 is inserted into the first puncture site p1 and various procedures are completed.

As illustrated in Fig. 17, the operator disposes the support member 200 on the dorsal side Hb of the right hand H1 of the patient. In this event, the operator can appropriately position the support member 200 at the first puncture site p1 by disposing the marker 315 at the first puncture site p1 while visually confirming the position of the marker 315 disposed at the inflatable portion 310.

Note that the operator may pull out part of the sheath tube 610 of the introducer 600 from the first puncture site p1 formed on the right hand H1 of the patient before attaching the hemostatic device 10 to the right hand H1 of the patient after finishing the procedure using the introducer 600. For example, in a state where the sheath tube 610 of the introducer 600 is indwelled in a blood vessel B, the operator pulls out the sheath tube 610 to the operator's hand side by about 2 to 3 cm, and then can start operation of attaching the hemostatic device 10.

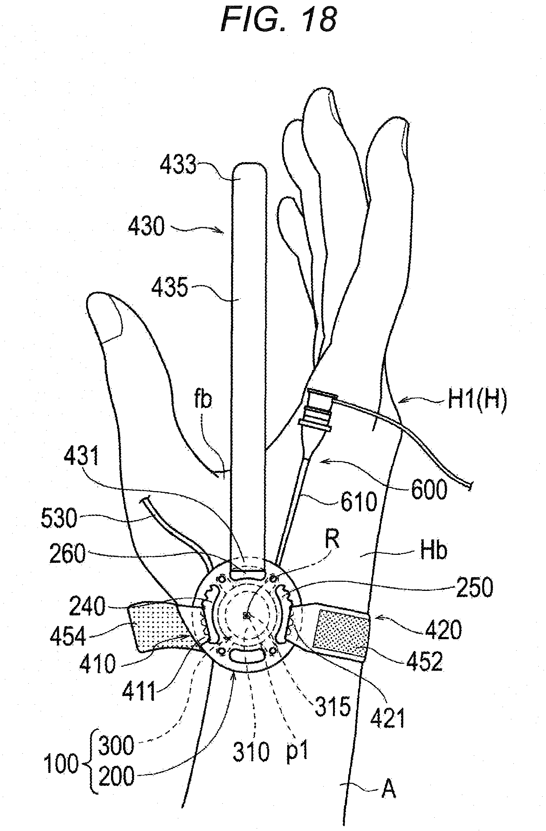

As illustrated in Figs. 17 and 18, the operator wraps the first band body 410 and the second band body 420 along the external periphery of the right hand H1 of the patient. The operator brings the fourth fixing portion 454 (see Fig. 2) disposed on the inner surface of the second band body 420 into contact with the first fixing portion 451 (see Fig. 1) disposed on the outer surface of the first band body 410, whereby the first band body 410 and the second band body 420 can be fixed via the fixing portions 451 and 454.

When the first band body 410 and the second band body 420 are wrapped along the external periphery of the right hand H1 of the patient, the operator can slide each of the band bodies 410 and 420 along each of the hole portions 240 and 250 (see Fig. 14). The operator can adjust the position around which each of the band bodies 410 and 420 is wrapped on the right hand H1 of the patient by sliding and moving each of the band bodies 410 and 420. For example, the operator can slide and move each of the band bodies 410 and 420 so that each of the band bodies 410 and 420 is wrapped around the right hand H1 of the patient at a position closer to the forearm A side (proximal side) than the first puncture site p1.

As illustrated in Fig. 19, the operator disposes part of the third band body 430 on the palm side of the right hand H1 of the patient while passing the third band body 430 through the interdigital part fb located between the thumb and the index finger of the right hand H1 of the patient. In this event, the operator brings the fifth fixing portion 455 (see Fig. 2) disposed on the inner surface of the third band body 430 into contact with the second fixing portion 452 (see Fig. 1) disposed on the outer surface of the second band body 420, so that the third band body 430 and the second band body 420 can be fixed via the fixing portions 452 and 455.

The operator disposes each of the band bodies 410 and 420 so as to be wrapped around the external periphery of the right hand H1 of the patient, and further disposes the third band body 430 by hooking part of the third band body 430 on the interdigital part fb between the thumb and the index finger of the right hand H1 of the patient, whereby the hemostatic device 10 can be effectively prevented from being displaced from the right hand H1 of the patient.

The operator inflates the inflatable portion 310 by injecting air into the inflatable portion 310 in a state where the syringe is connected to the injection portion 510.

As illustrated in Figs. 20 and 21, in the hemostatic device 10, if the inflatable portion 310 inflates, the inflatable portion 310 applies a compressive force to the first puncture site p1 of the right hand H1 of the patient.

In addition, in the hemostatic device 10, if the inflatable portion 310 inflates, the first band body 410 is pulled toward the second side surface 242 of the first hole portion 240 along with the inflating of the inflatable portion 310, and the second band body 420 is pulled toward the fourth side surface 254 of the second hole portion 250. In the hemostatic device 10, the protrusions 280 disposed on each of the side surfaces 242 and 254 bite into each of the band bodies 410 and 420. In the hemostatic device 10, if the protrusions 280 bite into each of the band bodies 410 and 420, at least one of both end portions located in the width direction of each of the band bodies 410 and 420 fits into the gap portions 245 and 255. The protrusion 280 closest to the end portion fitted into the gap portions 245 and 255 serves as a stopper that restricts sliding movement of each of the band bodies 410 and 420. As a result, the hemostatic device 10 exhibits a locking function by the protrusions 280 and restricts sliding movement of each of the band bodies 410 and 420 (see Fig. 15). Thus, the hemostatic device 10 can prevent each of the band bodies 410 and 420 from inadvertently sliding and moving while the inflatable portion 310 applies the compressive force to the first puncture site p1.

As illustrated in Figs. 19 and 20, when the hemostatic device 10 is attached to the right hand H1 of the patient, the operator can dispose the second curved region 232(see Figs. 8, 9, and 10) formed in the support member 200 along the external periphery of the right hand H1 of the patient. When the inflatable portion 310 inflates in a state where the hemostatic device 10 is disposed in this manner, the second curved region 232 of the support member 200 presses the inflatable portion 310 along part of the external periphery of the right hand H1 of the patient. Thus, the hemostatic device 10 can prevent the inflatable portion 310 from floating from the right hand H1 of the patient. Thereby, the hemostatic device 10 can effectively apply a compressive force to the first puncture site p1 by the inflatable portion 310.

As illustrated in Figs. 19 and 21, when the hemostatic device 10 is attached to the right hand H1 of the patient, the operator can dispose the first curved region 231(see Figs. 8, 9, and 10) formed in the support member 200 at the position on the distal side and the position on the proximal side of the right hand H1 of the patient. By disposing the hemostatic device 10 in this manner, when the patient twists the wrist and moves the right hand H1 in a vertical direction and a horizontal direction in a state where the hemostatic device 10 is attached to the right hand H1 of the patient, the first curved region 231 prevents a peripheral edge portion of the support member 200 from abutting on the right hand H1 of the patient. As a result, with the hemostatic device 10, it is possible to prevent the patient from feeling uncomfortable or feeling pain due to abutment or biting of the peripheral edge portion of the support member 200 against the right hand H1 of the patient while bleeding is stopped at the first puncture site p1 formed on the right hand H1 of the patient.

After the inflatable portion 310 inflates, the operator removes the sheath tube 610 of the introducer 600 from the first puncture site p1 as illustrated in Fig. 19. The operator confirms that there is no bleeding from the first puncture site p1 while bleeding is stopped using the hemostatic device 10. In a case where there is bleeding from the first puncture site p1, the operator adjusts an injection amount of air into the inflatable portion 310.

According to the above procedure, the operator can stop bleeding at the first puncture site p1 formed on the right hand H1 of the patient using the hemostatic device 10.

Fig. 22 illustrates a second use example of the hemostatic device 10. The second use example is a use example of the hemostatic device 10 when bleeding is stopped at the second puncture site p2 formed on the right hand H1 of the patient.

As illustrated in Fig. 22, when bleeding is stopped at the second puncture site p2 formed on the right hand H1 of the patient, the operator attaches the hemostatic device 10 to the right hand H1 of the patient. The second puncture site p2 formed on the right hand H1 of the patient is located on the distal side of the right hand H1 of the patient with respect to the first puncture site p1 described above (see Fig. 16). When each of the band bodies 410 and 420 is wrapped around the right hand H1 of the patient, the operator can slide and move each of the band bodies 410 and 420 along the extending direction of each of the hole portions 240 and 250. For example, the operator slides and moves each of the band bodies 410 and 420 so that each of the band bodies 410 and 420 is wrapped around the right hand H1 of the patient at a position closer to the forearm A side (proximal side) than the second puncture site p2. The operator can prevent the distal portion of the right hand H1 of the patient from being restrained by each of the band bodies 410 and 420 by wrapping each of the band bodies 410 and 420 around the position on the proximal side of the right hand H1 of the patient.

Although not illustrated, the hemostatic device 10 can also be used for stopping bleeding at a puncture site formed on the left hand of the patient. The puncture site formed on the left hand may be, for example, a puncture site formed on an artery located in a snuff box of a palmar artery running on the dorsal side of the left hand of the patient or a puncture site formed on a distal radial artery located on the distal side of the snuff box of the palmar artery running on the dorsal side of the left hand of the patient. The latter puncture site is located on the distal side of the left hand relative to the former puncture site with reference to the extensor pollicis longus tendon located on the dorsal side of the left hand of the patient.

Even in a case where bleeding is stopped at the puncture site of the left hand, the hemostatic device 10 can be attached to the left hand of the patient in the same procedure as in Use Example 1 and Use Example 2 described above. The operator can adjust the position at which each of the band bodies 410 and 420 is to be wrapped around the left hand by sliding and moving each of the band bodies 410 and 420 according to the position of each puncture site of the left hand of the patient. By adjusting the position at which each of the band bodies 410, 420 is to be wrapped around the left hand, the operator can prevent the portion on the distal side of the left hand of the patient from being restrained by each of the band bodies 410, 420 when bleeding is stopped at each puncture site.

As described through each use example, the hemostatic device 10 can be attached to either the right hand H1 or the left hand of the patient. In addition, even in a case where the hemostatic device 10 is used for stopping bleeding at the respective puncture sites p1 and p2 formed at different positions on the right hand H1 of the patient and in a case where the hemostatic device 10 is used for stopping bleeding at the respective puncture sites formed at different positions on the left hand of the patient, the hemostatic device 10 can be attached to the hand H of the patient so that the portion on the distal side of the hand H of the patient is not restrained by the respective band bodies 410 and 420.

### (Operational effects)

As described above, the hemostatic device 10 according to the present embodiment includes the pressing member 100 configured to compress the first puncture site p1 formed on the patient, the first band body 410 configured to be connectable to the pressing member 100, and the second band body 420 configured to be connectable to the pressing member 100. The pressing member 100 includes the support member 200 and the pressing portion 300 fixed to the support member 200 and configured to compress the first puncture site p1. The support member 200 includes the first region 210 in which the pressing portion 300 is disposed, and the second region 220 located outside the first region 210 and configured such that the first band body 410 and the second band body 420 are connectable. The second region 220 includes the first hole portion 240 configured such that the first band body 410 is connectable, and the second hole portion 250 located at a position facing the first hole portion 240 across the pressing portion 300 and configured such that the second band body 420 is connectable. The first hole portion 240 and the second hole portion 250 have a plurality of protrusions 280 protruding toward the pressing portion 300.

In the hemostatic device 10 configured as described above, the first band body 410 connected to the first hole portion 240 formed in the second region 220 of the support member 200 included in the pressing member 100 can slide and move along the first hole portion 240, and the second band body 420 connected to the second hole portion 250 formed in the second region 220 of the support member 200 can slide and move along the second hole portion 250. Thus, in the hemostatic device 10, the connection positions of the first band body 410 and the second band body 420 on the support member 200 can be easily adjusted when the hemostatic device 10 is attached to the hand H of the patient. As a result, the hemostatic device 10 has a high degree of freedom in the attachment position on the hand H of the patient. In addition, the first hole portion 240 and the second hole portion 250 formed in the support member 200 have a plurality of protrusions 280 protruding toward the pressing portion 300. When the hemostatic device 10 is attached to the hand H of the patient, the first band body 410 and the second band body 420 can be disposed to be wrapped along part of the hand H of the patient. If the first band body 410 and the second band body 420 are disposed so as to be wrapped around the hand H of the patient, a tensile force in a direction away from the pressing portion 300 is applied to the first band body 410 and the second band body 420 in the hemostatic device 10. As a result, the protrusions 280 positioned in the first hole portion 240 and the second hole portion 250 bite into the first band body 410 and the second band body 420. The protrusions 280 restrict sliding movement of the first band body 410 and the second band body 420 as a result of the protrusions 280 biting into the first band body 410 and the second band body 420. As a result, in the hemostatic device 10, it is possible to prevent the first band body 410 and the second band body 420 from inadvertently sliding and moving while the pressing portion 300 applies a compressive force to the first puncture site p1.

In addition, in the hemostatic device 10, the first hole portion 240 includes the first side surface 241, and the second side surface 242 which is located on a side farther away from the inflatable portion 310 than the first side surface 241 and which includes a plurality of protrusions 280. The second hole portion 250 has the third side surface 253 and the fourth side surface 254 which is located on a side farther away from the inflatable portion 310 than the third side surface 253 and includes a plurality of protrusions 280. The second side surface 242 and the fourth side surface 254 include the gap portions 245 and 255 formed between adjacent protrusions 280 among the plurality of protrusions 280.

In the hemostatic device 10 configured as described above, when each of the band bodies 410 and 420 is pressed against the protrusions 280, portions adjacent to portions in contact with the protrusions 280 in each of the band bodies 410 and 420 can be pushed into the gap portions 245 and 255. Thus, the hemostatic device 10 can cause the protrusions 280 to firmly bite into each of the band bodies 410 and 420. Thereby, the hemostatic device 10 can enhance the locking function of the protrusions 280.

In the hemostatic device 10, the protrusion 280 includes the central portion 281 including an apex of the protrusion 280 and connection portions 282 and 283 connecting the central portion 281 and the respective gap portions 245 and 255. Each of the connection portions 282 and 283 tilts from the central portion 281 toward each of the gap portions 245 and 255.

In the hemostatic device 10 configured as described above, the connection portions 282 and 283 tilt toward the respective gap portions 245 and 255, and thus, when each of the band bodies 410 and 420 is pressed against the protrusions 280, portions adjacent to portions in contact with the protrusions 280 in each of the band bodies 410 and 420 are easily guided along the connection portions 282 and 283 and pushed into the gap portions 245 and 255. Thus, the hemostatic device 10 can cause the protrusions 280 to bite into each of the band bodies 410 and 420 more firmly. Thereby, the hemostatic device 10 can enhance the locking function of the protrusions 280.

In addition, in the hemostatic device 10, at least part of the central portion 281 of each of the plurality of protrusions 280 located in the first hole portion 240 and at least part of the central portion 281 of each of the plurality of protrusions 280 located in the second hole portion 250 are located on the same virtual circle I.

In the hemostatic device 10 configured as described above, there is no variation in the position in the height direction (protruding direction) of the protrusions 280. Thus, the hemostatic device 10 can prevent each of the band bodies 410 and 420 from being caught by the protrusions 280 when each of the band bodies 410 and 420 slides and moves in a state where the protrusions 280 do not bite into each of the band bodies 410 and 420. Thus, in the hemostatic device 10, each of the band bodies 410 and 420 can smoothly slide and move.

In addition, in the hemostatic device 10, the protrusion 280 has a trapezoidal shape. The central portion 281 includes the linear flat portion 281a.

The hemostatic device 10 configured as described above is configured such that the central portion 281 includes the flat portion 281a, so that it is possible to prevent each of the band bodies 410 and 420 from being caught by the central portion 281 when each of the band bodies 410 and 420 slides and moves. Thus, each of the band bodies 410 and 420 can smoothly slide and move.

In the hemostatic device 10, the length L1 of each of the connection portions 282 and 283 is longer than the thickness D1 of the first band body 410 and the thickness D2 of the second band body 420.

In the hemostatic device 10, the length L1 of each of the connection portions 282 and 283 is formed to be longer than the thickness D1 of the first band body 410 and the thickness D2 of the second band body 420 as described above, and thus, when each of the band bodies 410 and 420 is pressed against the protrusions 280, part of each of the band bodies 410 and 420 can be pushed into the gap portions 245 and 255. Thus, the hemostatic device 10 can enhance the locking function of the protrusions 280.

In the hemostatic device 10, the width W2 of each of the gap portions 245 and 255 is smaller than the width W1 of the protrusion 280.

In the hemostatic device 10, the width W2 of the gap portions 245 and 255 is smaller than the width W1 of the protrusion 280 as described above, so that it is possible to prevent most of the band bodies 410 and 420 from entering the gap portions 245 and 255 in a state where the band bodies 410 and 420 are connected to the hole portions 240 and 250. Thus, in the hemostatic device 10, it is possible to prevent smooth sliding movement of each of the band bodies 410 and 420 from being hindered by most of the band bodies 410 and 420 entering the gap portions 245 and 255.

In the hemostatic device 10, the width W1 of the protrusion 280 is smaller than the width W31 of the first one end portion 411 of the first band body 410 and the width W32 of the second one end portion 421 of the second band body 420.

In the hemostatic device 10, the width W1 of the protrusion 280 is smaller than the width W31 of the first one end portion 411 of the first band body 410 and the width W32 of the second one end portion 421 of the second band body 420 as described above, so that, when each of the band bodies 410 and 420 is pressed against the protrusions 280, part of each of the one end portions 411 and 421 of each band body can be pushed into each of the gap portions 245 and 255. Thus, the hemostatic device 10 can enhance the locking function of the protrusions 280.

In the hemostatic device 10, the height h1 of the protrusion 280 from the first hole portion 240 side and the second hole portion 250 side toward the inflatable portion 310 side of the pressing portion 300 is larger than the thickness D1 of the first band body 410 and the thickness D2 of the second band body 420.

In the hemostatic device 10, the height h1 of the protrusion 280 is larger than the thickness D1 of the first band body 410 and the thickness D2 of the second band body 420 as described above, so that, when each of the band bodies 410 and 420 is pressed against the protrusions 280, part of each of the band bodies 410 and 420 can be pushed into the gap portions 245 and 255. Thus, the hemostatic device 10 can enhance the locking function of the protrusions 280.

The hemostatic device 10 includes the third band body 430 configured to be connectable to the pressing member 100. The second region 220 of the support member 200 has the third hole portion 260 located at a position different from the first hole portion 240 and the second hole portion 250 in the support member 200.

With the hemostatic device 10, the fixing force of the hemostatic device 10 to the hand H of the patient can be increased by fixing the hemostatic device 10 to the hand H of the patient using the third band body 430 connected to the third hole portion 260 located at a position different from the first hole portion 240 and the second hole portion 250 of the support member 200 together with the band bodies 410 and 420 connected to the support member 200. In addition, in a case where the hemostatic device 10 is used for stopping bleeding at the first puncture site p1 formed on the hand H of the patient, it is possible to more reliably fix the hemostatic device 10 to the hand H of the patient by disposing the third band body 430 in the interdigital part fb between the fingers while wrapping the first band body 410 and the second band body 420 along the external periphery of the hand H of the patient.

Next, modifications and other embodiments of the present invention will be described. In the following description, description of content overlapping with the content already described in the above-described embodiment will be omitted. Further, configurations, and the like, that are not particularly described in the following description can be the same as those of the above-described embodiment.

### <Modifications 1 to 4>

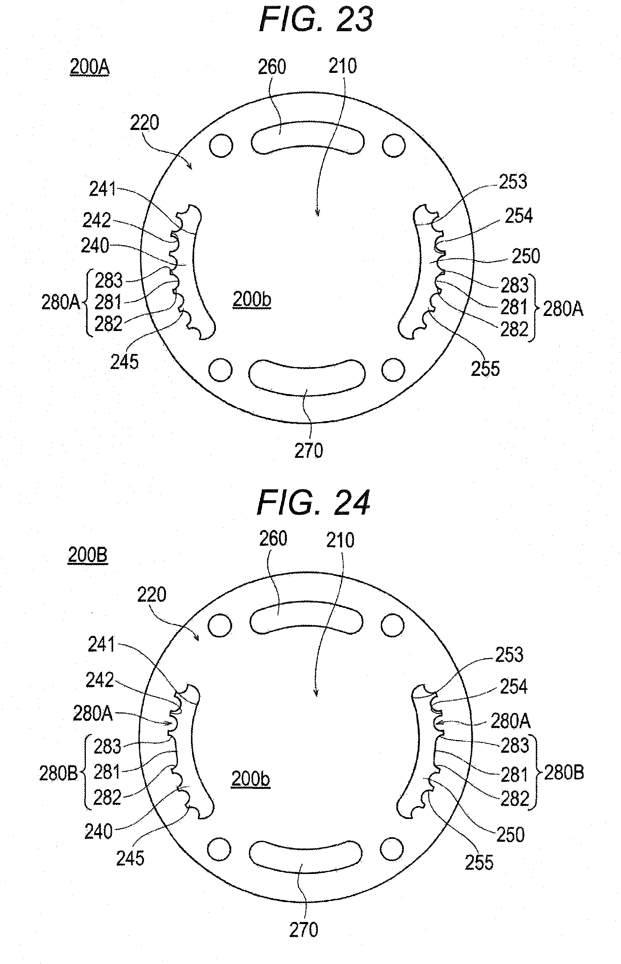

The shape of the protrusion formed on the support member 200 is not particularly limited as long as the protrusion has a locking function of preventing sliding movement of each of the band bodies 410 and 420. The protrusion can also be configured in a shape illustrated in Figs. 23 to 26, for example.

A protrusion 280A of a support member 200A according to Modification 1 illustrated in Fig. 23 has a shape in which a central portion 281 is curved. Connection portions 282 and 283 also have a curved shape. Thus, the protrusion 280A has a convex shape curved from the second side surface 242 toward the first side surface 241 and a convex shape curved from the fourth side surface 254 toward the third side surface 253.

In each of the hole portions 240 and 250 of a support member 200B according to Modification 2 illustrated in Fig. 24, a protrusion 280B in which the width of the central portion 281 is formed to be longer than the widths of the central portions 281 of the other protrusions 280A is disposed at a substantially central position in the extending direction of each of the hole portions 240 and 250. The protrusion 280B has a convex shape curved from the second side surface 242 toward the first side surface 241 and a convex shape curved from the fourth side surface 254 toward the third side surface 253, similarly to the protrusion 280A according to Modification 1.

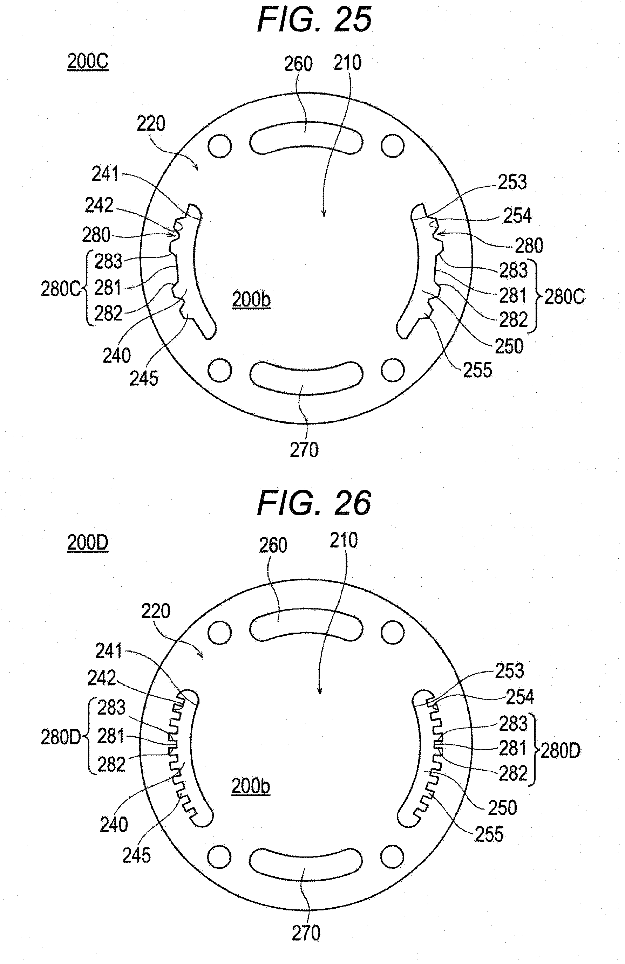

In each of the hole portions 240 and 250 of a support member 200C according to Modification 3 illustrated in Fig. 25, a protrusion 280C in which the width of the central portion 281 is formed to be longer than the widths of the central portions 281 of the other protrusions 280 is disposed at a substantially central position in the extending direction of each of the hole portions 240 and 250. The protrusion 280C has a trapezoidal shape having a flat portion 281a at the central portion 281.

In each of the hole portions 240 and 250 of a support member 200D according to Modification 4 illustrated in Fig. 26, a protrusion 280D in which an angle between the connection portions 282 and 283 and the central portion 281 is approximately 90° is formed. The protrusion 280D has a rectangular shape in plan view. The number of the protrusions 280D formed on the support member 200D according to Modification 4 is larger than the number of the protrusions 280 formed on the support member 200 according to the first embodiment (see Figs. 14 and 15).

When the support member 200 according to the first embodiment is compared with the support members 200A, 200B, 200C, and 200D according to the respective modifications, there is a difference in the following functional aspects due to a difference in the shape and the number of protrusions.

In the support member 200A according to Modification 1, the central portion 281 of the protrusion 280A is curved in a convex shape. Thus, a sliding function of each of the band bodies 410 and 420 is improved as compared with the support member 200. On the other hand, each of the band bodies 410 and 420 easily slides in a state where each of the band bodies 410 and 420 is in contact with the central portion 281 of the protrusion 280A, and thus, the locking function is lower than that of the support member 200.

The support member 200B according to Modification 2 includes the protrusion 280B whose central portion 281 has a curved convex shape formed over a wide range in the width direction. Thus, in the support member 200B, a sliding function of each of the band bodies 410 and 420 is improved as compared with the support member 200 A. On the other hand, each of the band bodies 410 and 420 easily slides in a state where each of the band bodies 410 and 420 is in contact with the central portion 281 of the protrusion 280B, and thus, the locking function is lower than that of the support member 200A.

The support member 200C according to Modification 3 includes the protrusion 280C having a flat portion 281a in which the central portion 281 is formed over a wide range in the width direction. Thus, the support member 200C has a locking function lower than that of the support member 200. However, the support member 200C has a locking function higher than that of the support member 200A according to Modification 1 and the support member 200B according to Modification 2 and has a sliding function higher than that of the support member 200D according to Modification 4 described below.

The support member 200D according to Modification 4 includes a large number of protrusions 280D formed in a rectangular shape. The protrusion 280D is formed in a rectangular shape, and thus, each of the band bodies 410 and 420 is easily caught by the protrusions 280D. Further, in the support member 200D, the attachment angle α (see Fig. 12A) formed between the first one end portion 411 of the first band body 410 in a state of being pressed against the protrusions 280 and each of the connection portions 282 and 283 is formed at an acute angle.

Thus, the support member 200D has a locking function higher than that of the other support members 200, 200A, 200B, and 200C.

As described above, in the hemostatic device 10, sliding performance and the locking function of each of the band bodies 410 and 420 can be adjusted according to the shape and the number of the protrusions disposed in each of the hole portions 240 and 250. For example, in comparison with the support members 200A, 200B, 200C, and 200D according to the modifications, the support member 200 including the protrusions 280 described in the first embodiment has a good balance between the sliding function and the locking function and has a specification with high usability.

### <Modification 5>

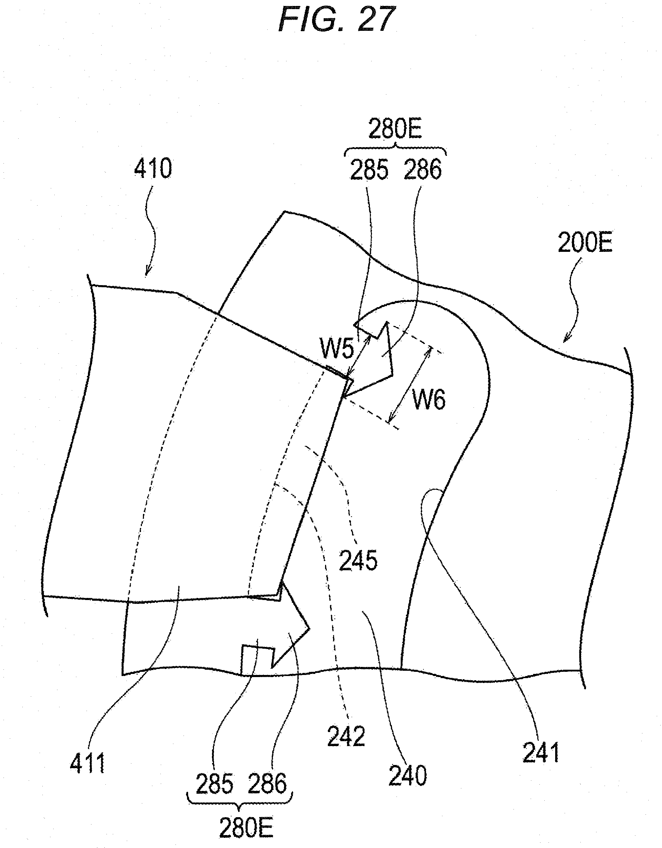

Fig. 27 illustrates a protrusion 280E according to Modification 5.

The protrusion 280E according to Modification 5 includes a base portion 285 extending toward the inflatable portion 310 of the pressing portion 300, and a locking portion 286 located on a side closer to the inflatable portion 310 of the pressing portion 300 than the base portion 285 and having a larger width than the base portion 285.

In Fig. 27, the "side closer to the inflatable portion 310 of the pressing portion 300" is a side on which the first side surface 241 is located.

The base portion 285 has a shape extending with a substantially constant width in plan view. The locking portion 286 has a triangular shape whose width gradually increases from the first side surface 241 side toward the second side surface 242 side.

A width W5 of the base portion 285 is smaller than a width W6 of the locking portion 286. Thus, a distance between the base portions 285 of the adjacent protrusions 280E is larger than a distance between the locking portions 286 of the adjacent protrusions 280E. Accordingly, as illustrated in Fig. 27, if the first band body 410 is inserted into the gap portion 245 formed between the base portions 285 through a gap formed between the locking portions 286, the first band body 410 is hooked on the locking portions 286. If the first band body 410 is disposed in this manner, the first band body 410 is less likely to come out of the gap portion 245, and thus, sliding movement of the first band body 410 can be more reliably restricted. In this manner, the locking function of restricting the sliding movement of the first band body 410 is further improved with the protrusions 280E including the base portions 285 and the locking portions 286.

Note that the protrusions 280E can be disposed at arbitrary positions of the first hole portion 240 and/or arbitrary positions of the second hole portion 250.

### <Second Embodiment>

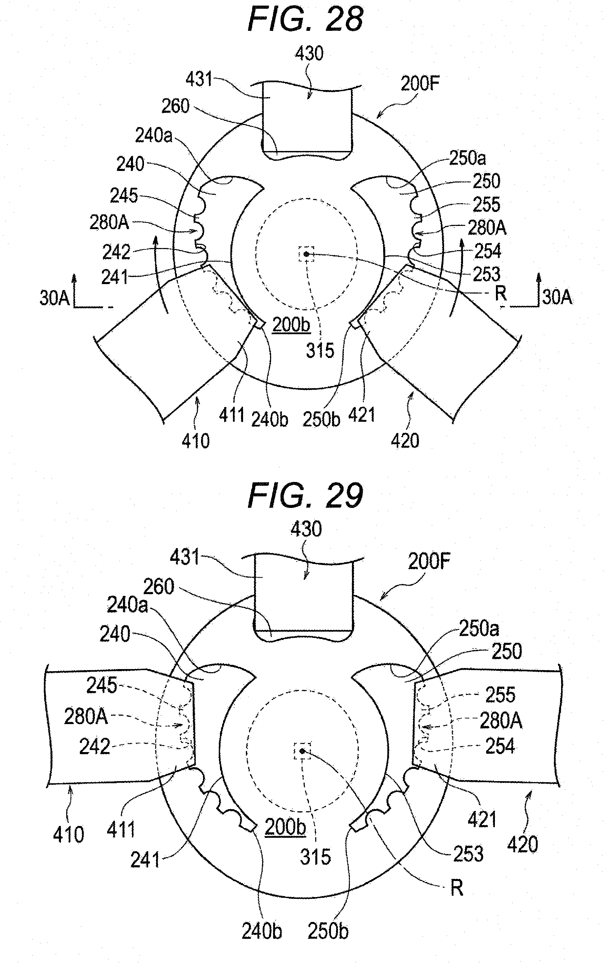

Figs. 28 and 29 illustrate a support member 200F according to a second embodiment.

As illustrated in Figs. 28 and 29, in the support member 200F according to the second embodiment, a distance between the first side surface 241 and the second side surface 242 of the first hole portion 240 and a distance between the third side surface 253 and the fourth side surface 254 of the second hole portion 250 gradually increase from the proximal portion side of the support member 400 toward the distal portion side of the support member 400.

The "proximal portion of the support member 200" is an end portion (end portion located on a lower side of Figs. 28 and 29) disposed on the forearm A side of the patient, for example, in a case where the hemostatic device 10 is attached to the hand H of the patient. In addition, the "distal portion of the support member 200" is an end portion (end portion located on an upper side of Figs. 28 and 29) disposed on the distal side of the forearm A of the patient, for example, in a case where the hemostatic device 10 is attached to the hand H of the patient.

The support member 200F has the protrusion 280A having the shape illustrated in Modification 1. However, a specific shape of the protrusion 280A is not particularly limited and may be, for example, the shape described in the first embodiment and other modifications.

In the present embodiment, the pressing portion 300 (inflatable portion 310) can be fixed to the one surface 200a of the support member 200F by adhesion or fusion, for example. Thus, the protruding portion 330 (see Fig. 11) is not attached to pressing portion 300.

A use example of the hemostatic device including the support member 200F according to the second embodiment will be described.

Fig. 30 is a cross-sectional view of the hemostatic device corresponding to an arrow 30A-30A illustrated in Fig. 28.

Fig. 30 illustrates a state in which the inflatable portion 310 inflates in a state in which the hemostatic device is attached to the hand H of the patient. In such a state, it is assumed that the band bodies 410 and 420 are disposed at the positions of the hole portions 240 and 250 illustrated in Fig. 28. For example, in a case where the operator desires to weaken the compressive force to be applied to the first puncture site p1 by the inflatable portion 310, the support member 200F is pressed against the hand H side as indicated by an arrow in Fig. 31 to weaken biting of the protrusions 280A with respect to each of the band bodies 410 and 420. The operator moves each of the band bodies 410 and 420 toward the one end portions 240a and 250a of each of the hole portions 240 and 250 (the distal portion side of the support member 200F) as indicated by an arrow in Fig. 28 in a state where biting of the protrusions 280A into each of the band bodies 410 and 420 is weakened. If the operator performs such operation, a distance between the first band body 410 and the second band body 420 increases as illustrated in Fig. 29. As a result, the fixing force for fixing the inflatable portion 310 to the hand H by each of the band bodies 410 and 420 is weakened, and thus, the compressive force to be applied to the first puncture site p1 by the inflatable portion 310 is reduced. In a case where the operator desires to increase the compressive force to be applied to the first puncture site p1 by the inflatable portion 310, the operator moves each of the band bodies 410 and 420 toward the other end portions 240b and 250b of each of the hole portions 240 and 250 (the proximal portion side of the support member 200F) in a state where biting of the protrusions 280A into each of the band bodies 410 and 420 is weakened.

As described above, in the support member 200F according to the present embodiment, the distance between the first side surface 241 and the second side surface 242 of the first hole portion 240 and the distance between the third side surface 253 and the fourth side surface 254 of the second hole portion 250 gradually increase from the proximal portion side of the support member 200F toward the distal portion side of the support member 200F.

According to the support member 200F configured as described above, by shifting the position where the first band body 410 is located in the first hole portion 240 and the position where the second band body 420 is located in the second hole portion 250 to the distal portion side or the proximal portion side of the support member 200F, a magnitude of the compressive force to be applied to the first puncture site p1 by the inflatable portion 310 can be easily adjusted without removing or rewinding each of the band bodies 410 and 420 wrapped around the hand H from the hand H.

Although the hemostatic device according to the present invention has been described above through the embodiments, the present invention is not limited only to the content described in the specification and can be appropriately changed based on the description of the claims.

In the description of the embodiments, the hemostatic device having a configuration in which the first band body and the second band body can slide and move with respect to the support member and the third band body cannot slide and move with respect to the support member has been exemplified. However, the hemostatic device may be configured such that the third band body can also slide and move with respect to the support member.

In addition, the configuration of the pressing portion that applies the compressive force to the puncture site is not limited to the inflatable portion (for example, a balloon-like structure). The pressing portion can be formed of, for example, a resin material such as plastic configured to be able to apply a compressive force to the puncture site, a member made of gel, or the like, an elastic material such as a sponge-like substance, an aggregate of fibers such as cotton (cotton), a metal, a member having a predetermined three-dimensional shape (spherical, ellipsoid, triangular pyramid, etc.), or a combination thereof as appropriate.

In the description of the embodiments, the hemostatic device for stopping bleeding at the puncture site formed on the dorsal side of the hand has been exemplified. However, the hemostatic device can also be used to stop bleeding at the puncture site formed on the palm of the hand. In addition, arrangement of each band body when the hemostatic device is attached to the patient is not limited to the position described by illustration, and can be appropriately changed. For example, the third band body can also be disposed at an interdigital part other than the interdigital part located between the thumb and the index finger. Similarly to the hand, the hemostatic device can also be used for stopping bleeding at a puncture site formed on the foot of the patient (for example, the dorsum pedis, the sole of the foot, and the like) having many moving parts such as fingers.

The shape, dimensions, and the like, of each portion of the hemostatic device are not particularly limited as long as an inflatable member can be disposed at a site where bleeding is to be stopped, and can be appropriately changed.

The present application is based on Japanese Patent Application No. 2022-006151 filed on January 19, 2022, the disclosure content of which is incorporated herein by reference in its entirety.

### Reference Signs List

10 Hemostatic device
100 Pressing member
200 Support member
200A Support member
200B Support member
200C Support member
200D Support member
200F Support member
200a One surface of support member
200b Other surface of support member
210 First region
220 Second region
240 First hole portion
240a One end portion of first hole portion
240b Other end portion of first hole portion
241 First side surface
242 Second side surface
245 Gap portion
250 Second hole portion
250a One end portion of second hole portion
250b Other end portion of second hole portion
253 Third side surface
254 Fourth side surface
255 Gap portion
260 Third hole portion
270 Fourth hole portion
280 Protrusion
280A Protrusion
280B Protrusion
280C Protrusion
280D Protrusion
280E Protrusion
281 Central portion
281a Flat portion
282 Connection portion
283 Connection portion
285 Base portion
286 Locking portion
290 Attachment hole
300 Pressing portion
310 Inflatable portion
310a Lumen of inflatable portion
315 Marker
330 Protruding portion
331 Flange portion
332 Connection portion
332a Through-hole
410 First band body
420 Second band body
430 Third band body
I Virtual circle
R Center point
S Space
L1 Length of connection portion
L2 Distance between protrusion and first side surface
L3 Distance between protrusion and second side surface
W1 Width of protrusion
W2 Width of gap portion
W31 Width of first one end portion (width of first band body)
W32 Width of second one end portion (width of second band body)
W41 Width of first hole portion
W42 Width of second hole portion
D1 Thickness of first band body
D2 Thickness of second band body
h1 Height of protrusion
α Attachment angle
A Forearm
B Blood vessel (artery)
H Hand
H1 Right hand
Hb Dorsal side of hand
fb Interdigital part
t1 Extensor pollicis longus tendon
p1 First puncture site (puncture site)
p2 Second puncture site (puncture site)

## Claims

1. A hemostatic device comprising:
a pressing member configured to compress a puncture site formed on a patient;
a first band body configured to be connectable to the pressing member; and
a second band body configured to be connectable to the pressing member, wherein
the pressing member includes a support member and a pressing portion fixed to the support member and configured to compress the puncture site,
the support member includes a first region in which the pressing portion is disposed, and a second region located outside the first region and configured such that the first band body and the second band body are connectable,
the second region includes a first hole portion configured such that the first band body is connectable, and a second hole portion located at a position facing the first hole portion across the pressing portion and configured such that the second band body is connectable, and
the first hole portion and the second hole portion have a plurality of protrusions protruding toward the pressing portion.

2. The hemostatic device according to claim 1, wherein
the first hole portion includes a first side surface, and a second side surface which is located on a side farther from the pressing portion than the first side surface and which includes the plurality of protrusions,
the second hole portion has a third side surface, and a fourth side surface which is located on a side farther away from the pressing portion than the third side surface and which includes the plurality of protrusions, and
each of the second side surface and the fourth side surface includes a gap portion formed between adjacent protrusions of the plurality of protrusions.

3. The hemostatic device according to claim 2, wherein
each of the protrusions includes a central portion including an apex of each of the protrusions and a connection portion connecting the central portion and the gap portion, and
the connection portion tilts from the central portion toward the gap portion.

4. The hemostatic device according to claim 3, wherein at least part of the central portion of each of the plurality of protrusions located in the first hole portion and at least part of the central portion of each of the plurality of protrusions located in the second hole portion are located on the same virtual circle.

5. The hemostatic device according to claim 3 or 4,
wherein
each of the protrusions has a trapezoidal shape, and
the central portion includes a linear flat portion.

6. The hemostatic device according to any one of claims 3 to 5, wherein a length of the connection portion is longer than a thickness of the first band body and a thickness of the second band body.

7. The hemostatic device according to any one of claims 2 to 6, wherein a width of the gap portion is smaller than a width of each of the protrusions.

8. The hemostatic device according to any one of claims 2 to 7, wherein a width of each of the protrusions is smaller than a width of the first band body and a width of the second band body.

9. The hemostatic device according to any one of claims 2 to 8, wherein a height of each of the protrusions from the first hole portion side and the second hole portion side toward the pressing portion side is larger than a thickness of the first band body and a thickness of the second band body.

10. The hemostatic device according to claim 2, wherein each of the protrusions includes a base portion extending toward the pressing portion, and a locking portion located closer to the pressing portion than the base portion and having a larger width than the base portion.

11. The hemostatic device according to any one of claims 2 to 10, wherein a distance between the first side surface and the second side surface and a distance between the third side surface and the fourth side surface gradually increase from a proximal portion side of the support member toward a distal portion side of the support member.

12. The hemostatic device according to any one of claims 1 to 11, comprising:
a third band body configured to be connectable to the pressing member,
wherein the second region has a third hole portion located at a position different from the first hole portion and the second hole portion in the support member.
